(19) **Europäisches Patentamt**

**European Patent Office**

**Office européen des brevets**

(11) **EP 1 385 570 B1**

(12) **EUROPEAN PATENT SPECIFICATION**

(45) Date of publication and mention
of the grant of the patent:
**13.09.2006 Bulletin 2006/37**

(51) Int Cl.:
*A61M 35/00* *(2006.01)*     *A61N 1/32* *(2006.01)*
*A61N 1/37* *(2006.01)*

(21) Application number: **02734124.7**

(86) International application number:
**PCT/US2002/013792**

(22) Date of filing: **30.04.2002**

(87) International publication number:
**WO 2002/087681 (07.11.2002 Gazette 2002/45)**

(54) **IMPLANTABLE MEDICAL DEVICE AND PATCH SYSTEM**

IMPLANTIERBARE MEDIZINISCHE VORRICHTUNG UND KISSENELEKTRODENSYSTEM

APPAREIL MEDICAL IMPLANTABLE ET SYSTEME DE TIMBRE

(84) Designated Contracting States:
**AT BE CH CY DE DK ES FI FR GB GR IE IT LI LU
MC NL PT SE TR**

(30) Priority: **30.04.2001 US 287521 P**

(43) Date of publication of application:
**04.02.2004 Bulletin 2004/06**

(73) Proprietor: **MEDTRONIC, INC.
Minneapolis, Minnesota 55432-5604 (US)**

(72) Inventors:
• **LAPORTE, Steve
Arden Hills, MN 55112 (US)**
• **HALLER, Markus
St. Louis, MN 55416 (US)**
• **HOOPER, William, J.
Lake Elmo, MN 55042 (US)**
• **LENT, Mark, S.
Brooklyn Park, MN 55433 (US)**
• **RIFF, Kenneth, M.
Orono, MN 55391 (US)**
• **HERUTH, Kenneth, T.
Edina, MN 55424 (US)**
• **OLSEN, James, M.
Plymouth, MN 55442 (US)**

(74) Representative: **Hughes, Andrea Michelle
Frank B. Dehn & Co.
St Bride's House
10 Salisbury Square
London EC4Y 8JD (GB)**

(56) References cited:
WO-A-01/28609          US-A- 4 655 766
US-A- 4 987 897          US-A- 5 551 953
US-A- 5 925 066

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

**Description**

RELATED APPLICATIONS

This application claims priority to provisional U.S. Application Ser. No. 60/287,521, filed April 30, 2001,

FIELD OF THE INVENTION

The present invention generally relates to medical devices. Specifically, the invention relates to a system, method and apparatus relating to a therapeutic substance delivery patch for attachment to a patient such that the patch is in contact with the patient's skin, wherein the patch coordinates its delivery of the therapeutic substance to the patient based on information received relating to the operation of an implanted device in the patient as defined in claim 1.

BACKGROUND OF THE INVENTION

Previously, a peripheral memory patch apparatus for attachment to a patient's skin has been disclosed. U.S. Patent No. 6,200,265 to Walsh et al., which is incorporated by reference herein, discloses such a memory patch including a high capacity memory for storing physiologic data uplinked from an implantable medical device.

Furthermore, earlier patents discuss various transdermal drug delivery devices. For example, U.S. Patent No. 5,135,480 to Bannon et al discloses a transdermal drug delivery device. More specifically, the Bannon invention relates to a transdermal device having a detachably mounted electrode with a first surface adapted for contact with human skin and through which a drug substance contained in the electrode passes to the skin under the influence of an iontophoretic or electro-osmotic force and a second surface which is electrically conducting, the electrode has a surface area in contact with the skin, in use, in the range 0.1 to 30 square centimeters and a drug dissolved or dispersed in a hydrophilic medium at a concentration in the range 0.1 to 15% (w/v) based on the hydrophilic medium.

Previous disclosures have also included the use of an implanted medical device as a network device including an ultimate result of delivering a drug to a patient. European Patent No. 1,022,035 to Arent discloses a network device that can be implanted in a subject. The device is configured to communicate over an internal wireless network or over an external computer network. In one embodiment, the device includes an internal interface in physiological communication with the subject. The internal interface is coupled with, and configured to send signals to a processor. The processor is configured to receive and process the signals, and is further configured to communicate over a computer network with another such device. Such devices can be used to monitor and communicate information regarding a host's physiological status, monitor and/or control natural and/or artificial organs, and prosthetic devices, and/or dispense medication. Drug delivery is accomplished through a separate drug reservoir and intravenous line or through another implanted device.

As can be seen by the above prior art, therapeutic substance therapy in conjunction with the activities and information obtained by an implanted medical device is an important consideration in the overall treatment of a patient. However, there is a need for a system, apparatus and method wherein a device is affixed to the body in contact with the skin, wherein such device delivers a therapeutic substance to the patient while allowing the patient to remain mobile. While mobility might be achieved by a second implanted drug delivery device, it is desired to avoid the complications and invasiveness of a second implanted device for the storage and delivery of the therapeutic substance.

WO-A-01-28609 discloses a drug delivery system where an external transdermal drug delivery device communicates with an implanted cardiac device via a radio frequency link ie. telemetry which controls release of drug. The drug delivery device can also react on a physiological effect of drug delivery.

BRIEF DESCRIPTION OF THE DRAWINGS

FIG. 1 is a schematic of one embodiment of the system according to the principles of the invention;

FIG. 2 is a schematic of one embodiment of a therapeutic substance delivery patch according to the principles of the invention;

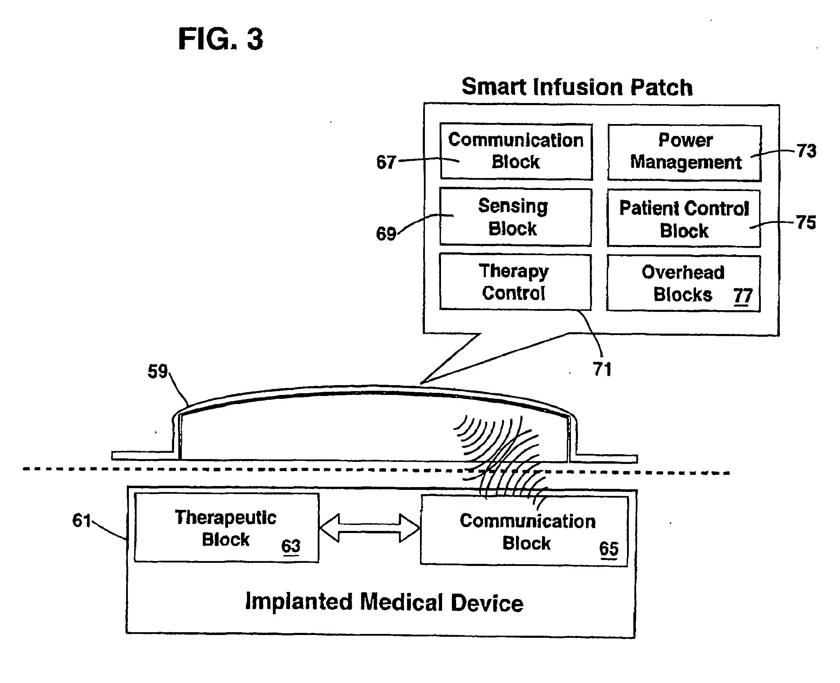

FIG. 3 is a schematic of one embodiment of the system according to the invention;

FIG. 4 is a top perspective partial cutaway view of another embodiment patch according to the principles of the invention;

FIG. 5 is a bottom perspective view of the embodiment patch of FIG. 4;

FIG. 6 is a top perspective view of the embodiment patch of FIGS. 4 and 5;

FIG. 7 shows a permanent magnet solenoid pump of the embodiment patch shown in FIG. 4;

FIG. 8 shows an exploded view of the permanent magnet solenoid pump of FIG. 7 embodiment;

FIG. 9 shows an exploded view of a pump piston for a permanent magnet solenoid pump three-coil embodiment for the patch embodiment of FIG. 4;

FIG. 10 shows an isometric cross-section view of a pump piston for a permanent magnet solenoid pump three-coil embodiment for the patch embodiment of FIG. 4;

FIG. 11 shows a cross section view of a pump piston for a permanent magnet solenoid pump three-coil embodiment for the patch embodiment of FIG. 4;

FIG. 12 shows an isometric cross-section view of a permanent magnet solenoid pump two-coil embodiment for the patch embodiment of FIG. 4;

FIG. 13 shows a cross section view of a permanent magnet solenoid pump two-coil embodiment for the patch embodiment of FIG. 4;

FIGS. 14a and 14b show a pump piston with an alternative piston fluid path embodiments for the patch embodiment of FIG. 4;

FIG. 15 shows an outlet valve embodiment for the patch embodiment of FIG. 4;

FIG. 16 shows a schematic of a two-coil permanent magnet solenoid pump embodiment for the patch embodiment of FIG. 4;

FIG. 17 shows a schematic of a two-coil with permanent magnet pole pieces permanent magnet solenoid pump embodiment for the patch embodiment of FIG. 4;

FIG. 18 shows a schematic of a three-coil permanent magnet solenoid pump embodiment for the patch embodiment of FIG. 4;

FIG. 19 shows a schematic of a three-coil with permanent magnet pole pieces permanent magnet solenoid pump embodiment for the patch embodiment of FIG. 4;

FIG. 20 shows a flow diagram of a method for operating a permanent magnet solenoid therapeutic substance delivery device embodiment for the patch embodiment of FIG. 4.

FIG. 21 represents another embodiment patch in bi-directional communications with an implanted medical device;

FIG. 22 shows an external programmer or a remote home device such as used to uplink and downlink data with both patch and the implanted medical device for the embodiment of FIG. 21;

FIG. 23 represents one aspect of the remote communication and monitoring system of the FIG. 21 embodiment;

FIG. 24 is a schematic block diagram of an automatic atrial cardioverter and pain alleviating system of the FIG. 21 embodiment of the present invention employing the automatic delivery of pain alleviating drug therapy;

FIG. 25 illustrates another representative therapeutic substance delivery patch;

FIG. 26 is a schematic drug delivery system block diagram;

FIG. 27 represents a high-level logic flow diagram of the FIG. 21 embodiment of the present invention.

The embodiments where drug delivery is based solely on telemetry do not fall under the invention.

DETAILED DESCRIPTION OF THE INVENTION

Preferred embodiments of this invention provide a apparatus or system in which a smart externalized infusion patch attached to the skin of a patient receives information from an implantable medical device and infuses a therapeutic substance based on the information.

A patch is defined as a device configured to be external to the body, but also including the possibility of being partly internal to the body (for example a patch may include a needle that protrudes through the skin), wherein the device is configured to be in contact with the skin of the patient. For example, a patch may be adhered to the skin of the patient, it may be maintained in contact via a strap such as with a wristwatch, it may be a ring worn around a patient's finger, or it may be attached via a larger strap worn around a patient's torso or other body part.

The patch may be attached to the skin of a patient at any location on the patient where the patch can receive the necessary information to make a determination to deliver a therapeutic substance to the patient and advantageously deliver the therapeutic substance. In a preferred embodiment the patch is near the location of the implanted device to maximize ability to best receive the information. However, any patch location is acceptable as long as the patch is capable of receiving the information.

Via this remote interaction, the external patch is able to read cardiac, neurological or other physiological patient data that is accessed by the implanted medical device, and program the external patch for therapeutic substance delivery. Among events may be therapies that combine drug administration and electrical stimulation for pain, atrial fibrillation (AF), heart failure (HF), Parkinson disease, epilepsy, etc. by continually or periodically obtaining physiologic input from the implanted device and then subsequently controlling the delivery of solutions that have either narrow therapeutic indices, strict patient compliance requirements, or rapid delivery versus event detection requirements (e.g., ischemia).

Various preferred embodiments of this system and method may be used for delivery of anti-arrhythmic drugs, diuretic drugs, vasodilators, muscle relaxants, pain drugs, anti-clotting drugs, blood thinners, anti-seizure drugs, naturitic peptides, insulin and any other therapeutic substance whose administration would be improved if the delivery could be optimally timed to the receipt of physiological information from an implantable medical device.

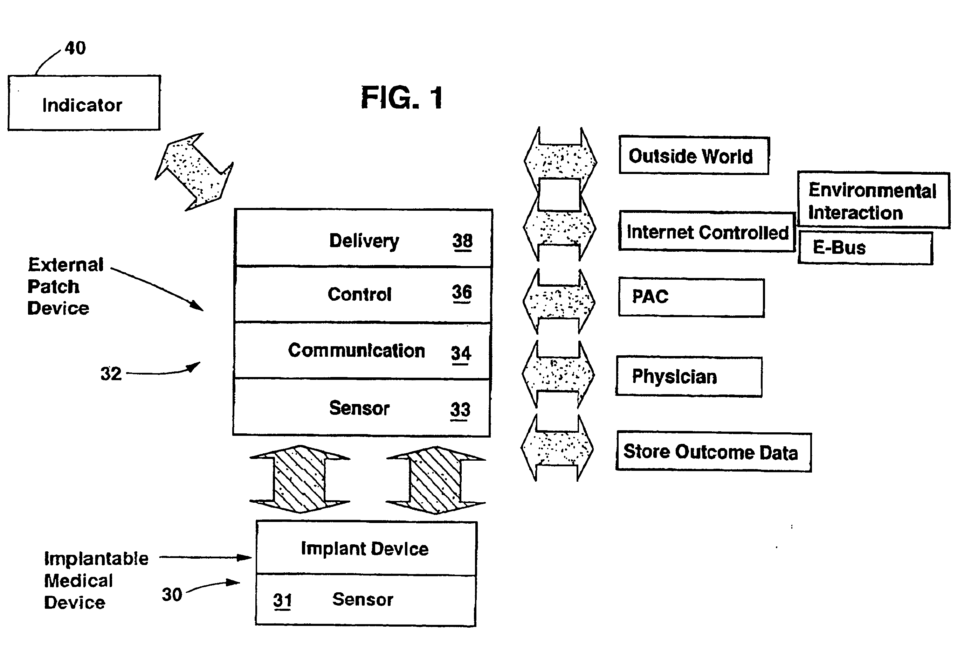

FIG. 1 is a block diagram of a medical therapy system including an implanted medical device 30 and a patch 32 for attachment to the skin of a patient. The implanted medical device (IMD) 30 may be any implanted medical device for providing a therapy to a patient or for monitoring physiological information about a patient or for providing both therapy and monitoring functions. For example, IMD 30 may be any of the following, but is not limited to a pacemaker, defibrillator, cardioverter, pacemaker/cardioverter/defibrillator (PCD), oxygen sensing device, nerve stimulator, brain stimulator, muscle stimulator, infusion pump, drug delivery device, monitoring device, left ventricular assist device or total artificial heart device.

The IMD 30 may include communication capabilities for communicating with the patch 32. Such communication capabilities may include telemetry or any other medical device communication system.

IMD 30 may also include a sensor 31 for sensing a physiological condition of the patient. The sensor 31 may sense temperature; oxygen saturation; blood pressure; activity level, lung wetness, glucose level, impedence, EKG, heart rate, respiration rate, EMG, EEG, position of the patient and any host of other physiologic conditions that are relevant to the delivery of a therapeutic substance such as a drug. Many different types of sensors for sensing the above conditions are known in the art.

Patch 32 includes a communication module 34, control module 36 and a delivery module 38. Patch 32 include a sensor 33 whereby the patch 32 may obtain information regarding the implanted device and/or physiologic condition by sensing as opposed to by telemetry, or in combination with telemetric information. Patch 32 is configured for attachment to the patient's body such that the patch is in contact with the skin of the patient to provide for mobility. Attachment of the patch 32 to the skin of the patient may be through adhesives, straps, or any other means known.

Communication module 34 provides telemetry or other communication capabilities that allow the patch 32 to communicate with the IMD 30.

Control module 36 functions to control the operation of the patch 32 including control of the therapeutic substance delivery functions.

Delivery module 38 includes a reservoir for holding the therapeutic substance and therapeutic substance delivery means for providing the therapeutic substance to the patient in an appropriate amount.

The patch 32 may optionally include the capability to communicate with systems outside the patient. For example, the patch 32 may communicate with a network that in turn communicates with the Internet. Physicians, patients and others may then access any information deemed useful. Furthermore, data may be downloaded for storage and review.

The patch 32 may optionally include the capability to provide an indication to the patient, doctor or others regarding the condition of the patient through indicator 40. Indicator 40 may reside on the patch 32 or it may be located elsewhere and communicate with the patch through telemetry or any other medical device communication means.

One embodiment of the invention may be used in a therapy for administering a therapeutic substance to a patient having an implanted medical device. The method includes attaching a patch 32 containing the therapeutic substance to the patient's body such that the patch is in contact with the skin of the patient. The method further includes generation of a signal from the IMD 30. In response to the signal generated by the medical device, the patch then administers the therapeutic substance to the patient.

Numerous, more specific, example therapies of the above embodiment of the invention are provided below. It is noted however, that the inclusion of these examples is not meant to be limiting to the scope of the present invention.

For the disease state of hyper or hypotension the likely parameter sensed by the implanted medical device is blood pressure. Blood pressure may be monitored by the implanted Chronicle® hemodynamic monitor sold by Medtronic, Inc. Other blood pressure monitors may also be used.

When high blood pressure is sensed by the implanted monitor or other device, such information is sent to the external patch device by telemetry or other wireless communication. The external patch device determines whether to provide a therapeutic substance to the patient and how much therapeutic substance to provide.

Some drugs that may be used to treat hyper or hypotension include diuretic drugs such as Furosemide and Bumentanide; beta blockers such as Propranolol and Metaprolol; and vasodilators such as Nitroglycerine and Isosorbide. However, the scope of this invention is not limited by the use of the drugs listed here. Certainly any drugs that have a therapeutic effect on hyper or hypotension, or that have an effect on any negative effects of the treatment by the implanted device, or that are developed in the future, are contemplated for use by this invention.

For the disease state of epilepsy and the associated seizures, the likely parameter sensed by the implanted medical device is the electric potentials of the brain, which is oftentimes recorded in an electroencephalogram (EEG). Electric potential of the brain may be measured by electrodes attached to the scalp or in other locations near the brain. Electric potentials in the brain may also be measured by deep brain stimulation leads (DBS leads) or cortical surface electrodes. In this case the likely implanted medical device is a neurostimulator. However, other implanted devices that have the capability to sense electric potentials of the brain may be used.

When seizure activity is anticipated, predicted or detected by the implanted medical device such information is sent to the external patch device by telemetry or other wireless communication. The external patch device determines whether to provide a drug to the patient and how much drug to provide.

Some drugs that may be used for epilepsy therapy include Phenytoin and Conantonkin-G. However, the scope of this invention is not limited by the use of the drugs listed here. Certainly any drugs that have a therapeutic effect on epileptic seizure activity, or that have an effect on any negative effects of the treatment by the implanted device, or that are developed in the future are contemplated for use by this invention.

For the disease state of diabetes, including hyperglycemia and hypoglycemia, the likely parameter sensed is glucose level in the blood or tissue. Glucose level may be measured through many well known methods and devices including, for example, the use of an implanted glucose monitor.

When the implanted glucose monitor senses hyperglycemia, the implanted glucose monitor sends such information to the external patch device by telemetry or other wireless communication. The external patch device determines whether to provide insulin or glucagons to the patient. For the case of hyperglycemia the external patch device delivers insulin to the patient. For the case of hypoglycemia the external patch device delivers glucagons to the patient.

For the disease state of ischemic heart disease, the likely parameter sensed is heart rate or arrythmias. Heart rate may be sensed, for example, by a pacemaker lead.

When the implanted pacemaker senses a heart rate above a certain point, the pacemaker sends such information to the external patch by telemetry or other wireless communication. The external patch determines whether to provide nitrates/BNP, biologics or peptides to the patient.

For the disease state of myocardial infarction, the likely parameter sensed by the implanted medical device is abnormalities in the EKG. EKG may be sensed by many methods and devices known including, for example, an implanted pacemaker or sensor such as a Reveal™ implanted loop recorder.

When the implanted pacemaker or monitor senses abnormalities in the EKG, the implanted device sends such information to the external patch by telemetry or other wireless communication. The external patch determines whether to provide an anticoagulant such as heparin or Tissue Plasminogen Activator (TPA) or an anti-arrythmic drug to the patient.

The invention may be used in, a method of administering a therapeutic substance to a patient in response to a sensed reading of the patient's physiologic reaction to an action taken by an implanted device is provided. The method includes attaching a patch containing the therapeutic substance to the patient such that the patch is in contact with the skin of the patient and a sensor for sensing a physiologic reaction to the action taken by the medical device. The method further includes sensing with the sensor the physiologic reaction to the action taken by the implanted medical device. The method also includes administering the therapeutic substance to the patient in response to the reaction sensed by the sensor.

A more specific example of the above alternative embodiment therapy relates to respiratory depression in response to delivery of morphine. In this example, the implanted medical device is an implanted drug pump such as the Synchromed® implantable drug pump made by Medtronic, Inc. The implanted drug pump may be used to deliver an opioid, such as morphine, fentanyl, sufentanil, and hydromorphone, to the spinal cord. Patients taking morphine may experience respiratory depression as a side effect. Therefore, if the patch senses a respiration rate that is lower than normal, it may respond by delivering a narcotic antagonist to the patient. The narcotic antagonist counters the effects of the morphine or other opioid. An example narcotic antagonist is naloxone. Respiration depression may also be sensed by the patch by sensing parameters such as heart rate, temperature, activity, galvanic response, etc. Note alternatively that respiration rate could also be sensed directly by the IMD and communicated to the patch

Another example therapy in which the patch senses the physiologic reaction to the action taken by the IMD relates to bupivicaine drug delivery. In the example of bupivicaine drug delivery by an implanted drug pump, cardiotoxicity can occur resulting in a higher, then a very low heart rate. The patch could detect those higher and then lower heart rates and infuse a vasodilator such as epinephrine.

The invention may be used in a therapy for administering a therapeutic substance to a patient having an implanted medical device. The method includes attaching a patch 32 containing a therapeutic substance, the patch including a sensor 33, to the patient such that the patch is in contact with the skin of the patient. The method further includes sensing with the sensor the action taken by the implanted medical device and administering the therapeutic substance to the patient in response to the action sensed by the sensor.

The invention may be used in a therapy for administering a therapeutic substance to a patient having an implanted medical device that is adapted to take an action on the patient. The method includes attaching a patch containing the therapeutic substance to the patient such that the patch is in contact with the skin of the patient. The patch includes a sensor for determining if the medical device has taken an action on the patient. The method further includes determining with the sensor whether the medical device has taken an action on the patient. The method further includes administering the therapeutic substance to the patient in response to the determination carried out in the determining step.

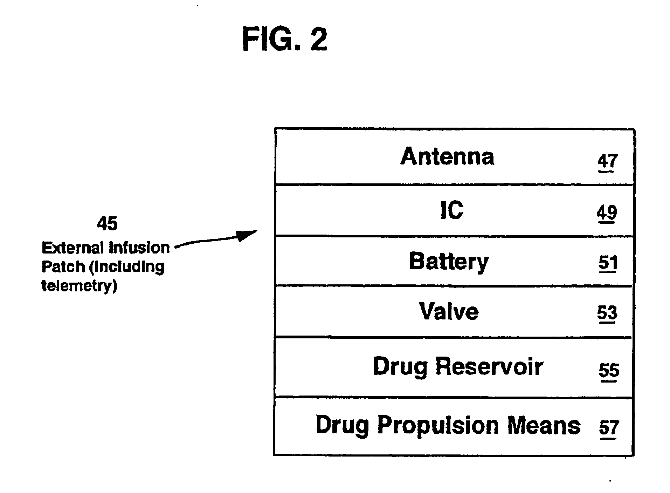

One embodiment of therapeutic substance delivery patch 32 is patch 45 shown in FIG. 2. Patch 45 includes an antenna 47 for telemetry applications, an integrated circuit 49, battery 51, valve 53, drug reservoir 55 and drug propulsion means 57.

One embodiment system includes patch 59 and implanted medical device 61. Implanted medical device 61 includes a therapeutic block 63 and communication block 65. The therapeutic block 63 is configured to provide some therapy to the patient's body. Patch 59 includes a communication block 67 for communicating with the implanted medical device 61, sensing block 69 including a sensor for the capability of sensing conditions in the body, therapy control block 71, power management block 73 for managing the power resources on board the patch, patient control block 75, and overhead blocks 77.

Various means for delivering the therapeutic substance from the reservoir on the patch to the patient are considered for this invention including, but not limited to, hypodermic needle, implanted catheter access port and iontophoresis.

It is also noted that the patch of the invention or a portion of the patch may be disposable. For example, the therapeutic substance reservoir could be disposable and replaced with a new reservoir module after the therapeutic substance is used.

**Detailed Exemplary Embodiment Patch (Patch 130)**

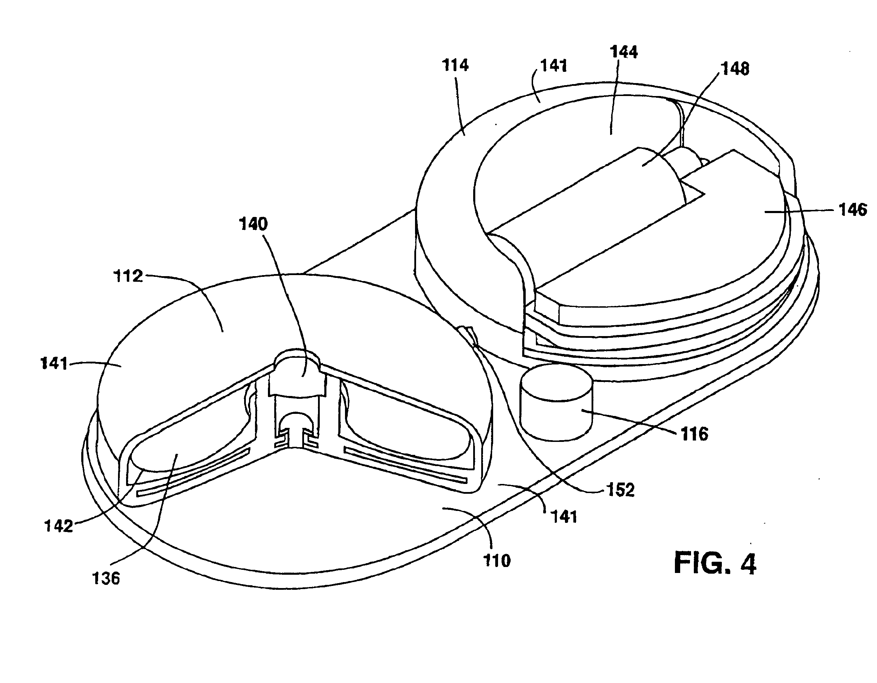

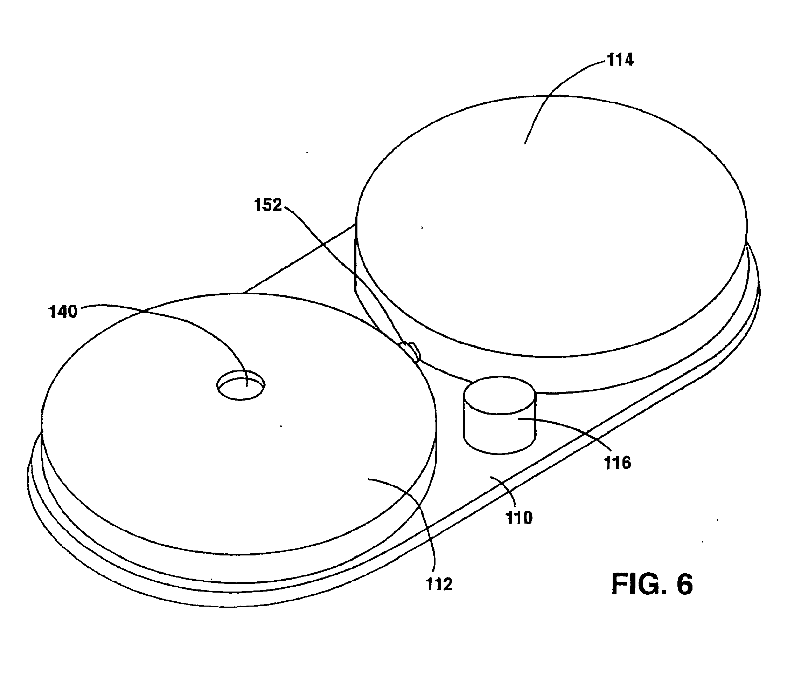

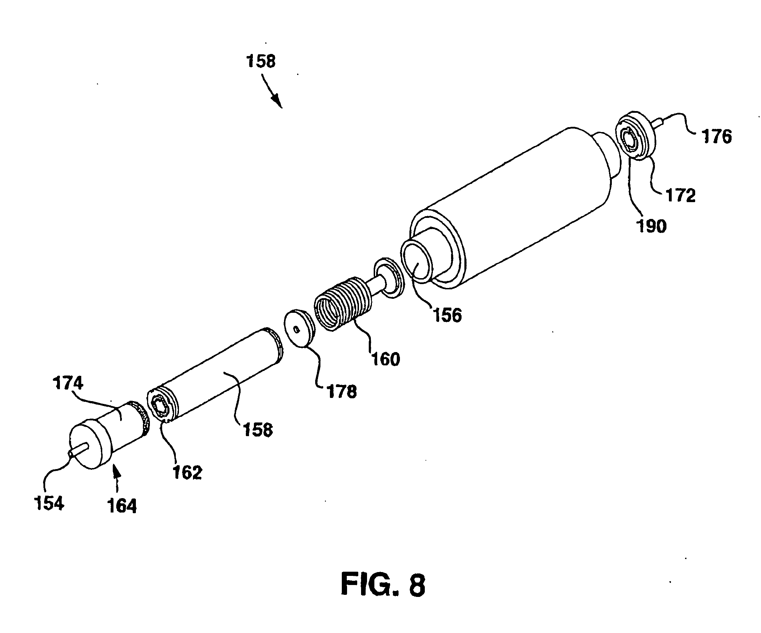

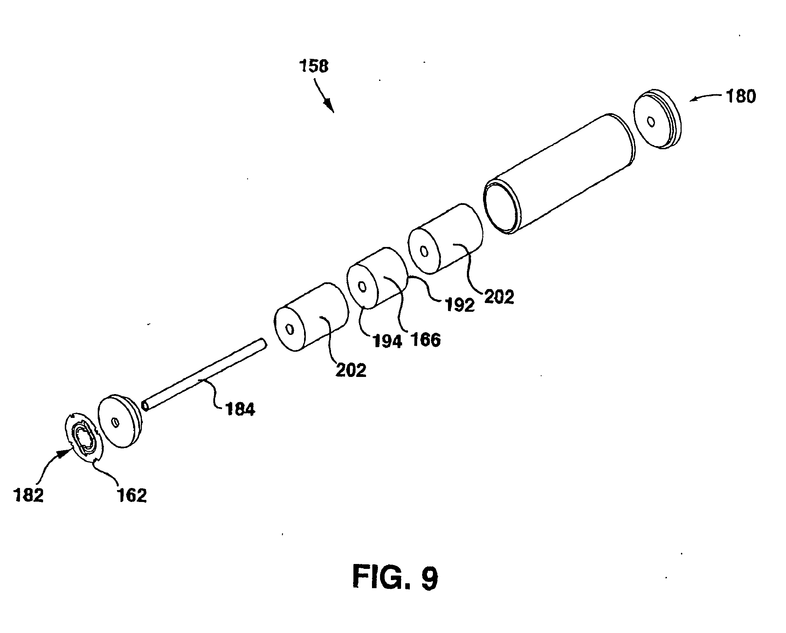

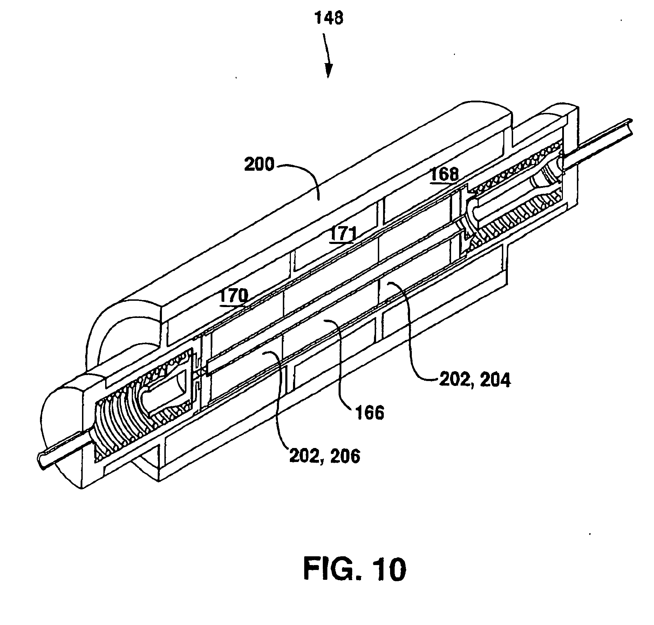

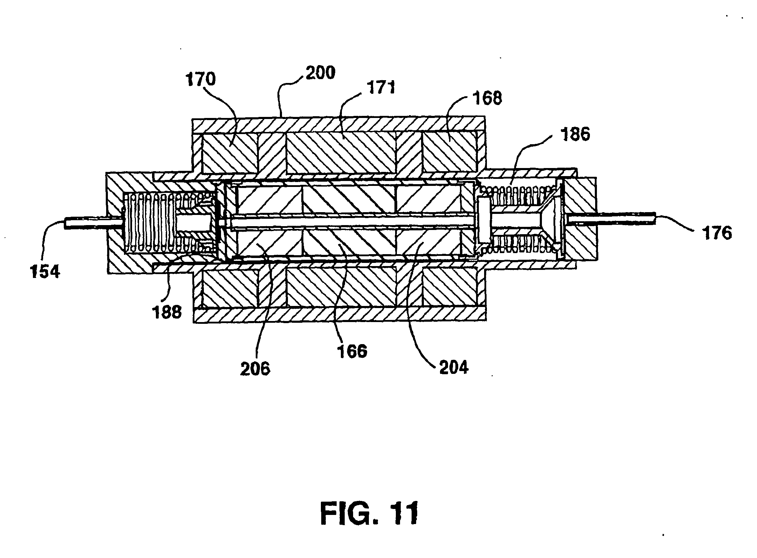

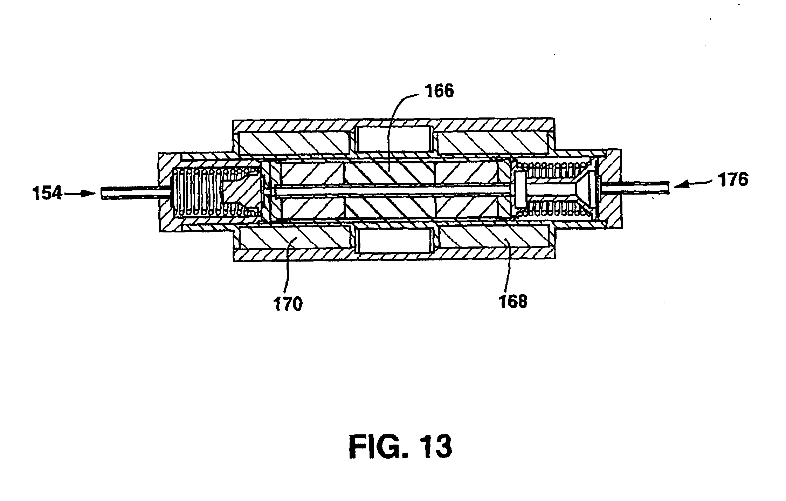

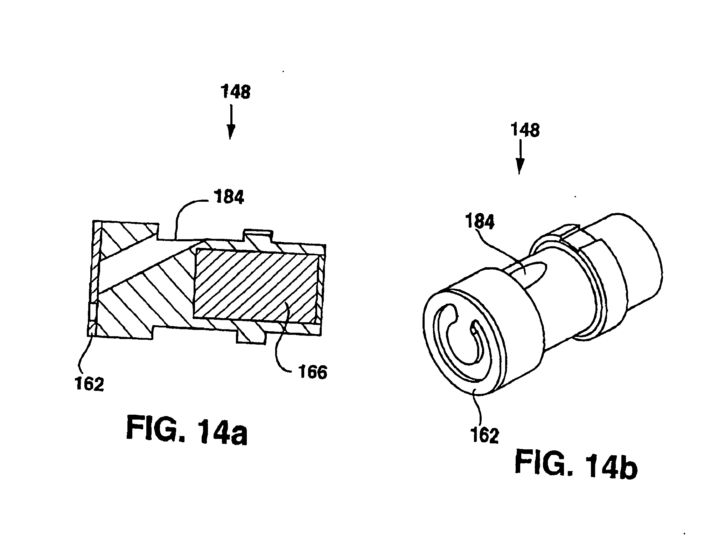

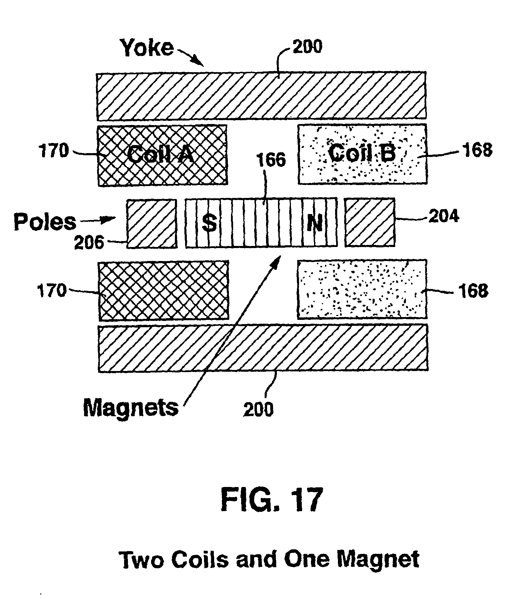

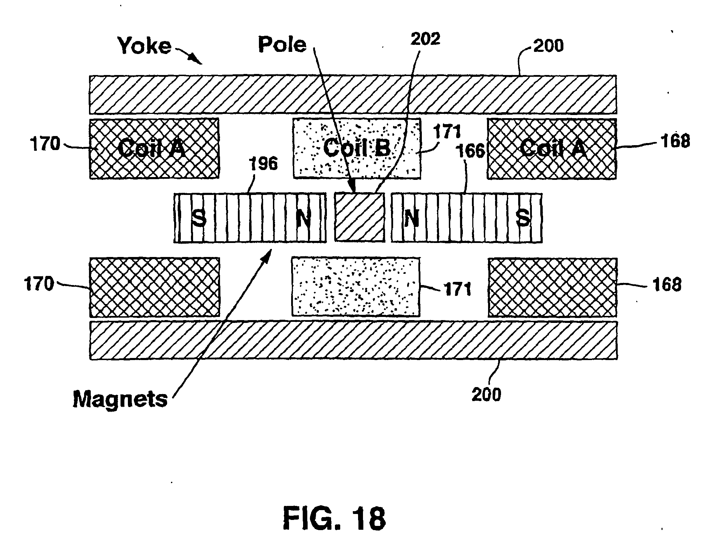

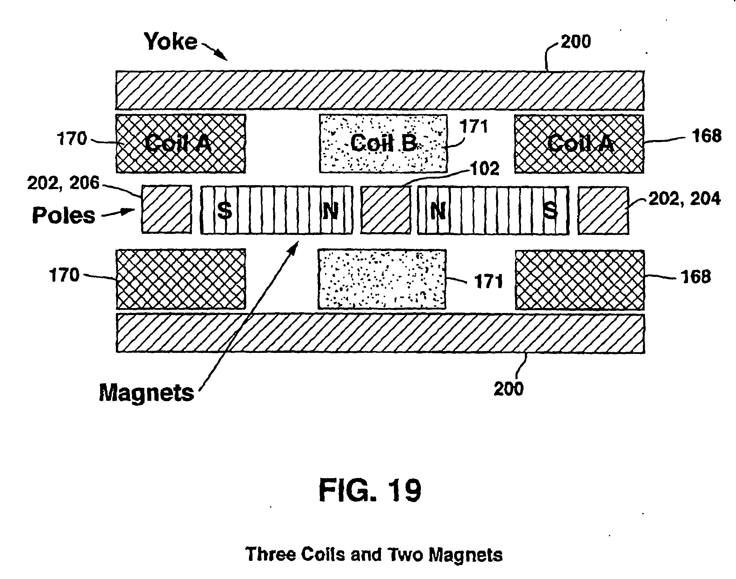

Another embodiment of patch 32 of the present invention will now be described in conjunction with FIGS. 4-20. FIG. 4 shows a therapeutic substance delivery patch 130. Patch 130 includes a housing 141, therapeutic substance reservoir 142, a power source 144, electronics 146, and a permanent magnet solenoid pump 148.

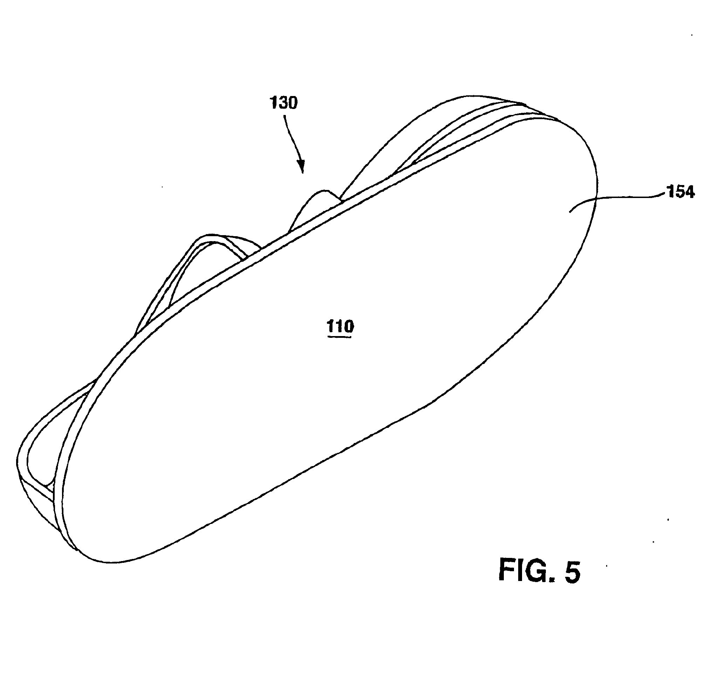

The above components of the patch may be contained within a single housing member or may be provided in a two member housing such as shown in FIG. 4. In the embodiment of FIG. 4, housing 141 includes a base portion 110 for positioning adjacent the skin of the patient (see FIG. 5), as well as two upper housing members 112 and 114.

Base portion 110 is designed to be positioned adjacent the exterior of the patient's skin surface. Base portion 110 may be flat or it may be curved somewhat to fit along the curvatures of the patient's body at the point of application.

In one embodiment, base portion 110 includes an adhesive on the side adjacent the skin surface for attaching the patch to the patient's skin. Other means for attaching the patch 130 may be utilized such as a strap or belt.

The upper housing member 112 forms the reservoir 142 configured for containing a therapeutic substance 136 and may use geometries such as metal bellows, polymeric bag, and the like. The therapeutic substance reservoir 142 has a reservoir outlet 152 and can have a septum 140 for refilling the reservoir 142. The patch 130 operates to infuse the therapeutic substance 136 at a programmed rate into a patient.

A therapeutic substance 136 is a product or substance intended to have a therapeutic effect such as pharmaceutical compositions, genetic materials, biologics, and other substances. Pharmaceutical compositions are chemical formulations intended to have a therapeutic effect such as antispasmodics, analgesics, pain medications, chemotherapeutic agents, and the like. Genetic materials are substances intended to have a direct or indirect genetic therapeutic effect such as genetic vectors, genetic regulator elements, genetic structural elements, DNA, and the like. Biologics are substances that are-living matter or derived from living matter intended to have a therapeutic effect such as stem cells, platelets, hormones, biologically produced chemicals, and the like. Other substances are substances intended to have a therapeutic effect yet are not easily classified such as saline solution, fluoroscopy agents, and the like.

The power source 144 is carried in the housing member 114 of housing 141. The power source 144 is selected to operate the solenoid pump 148 and electronics 146 such as a lithium ion (Li+) battery, capacitor, and the like. The electronics 146 are coupled to the power source 144 and typically include memory and a controller. The controller can be an Application Specific Integrated Circuit (ASIC) state machine, a gate array, or may include a microprocessor. The electronics 146 are configured to control the solenoid pump 148 infusion rate and can be configured to operate many other features such as patient alarms and the like. The electronics 146 can also include telemetry circuitry configured to receive and send information when the therapeutic substance delivery device 130 is implanted to allow programming of the infusion rate. In this embodiment, the telemetry antenna 116 is positioned on the base portion 110. The solenoid pump 148 is coupled to the electronics 146 and coupled to the therapeutic substance reservoir outlet 152 and configured for pumping therapeutic substance 136 from the therapeutic substance reservoir 142 through an infusion needle 154 at a programmed rate.

Infusion needle 154 is configured for insertion through a patient's skin for delivery of the therapeutic substance into the body. In one implementation of the therapy, the patch 130 may be attached to the patient's skin for about 4-8 days with the needle inserted through the skin. Various methods known in the art may be utilized to prevent infection with such a system.

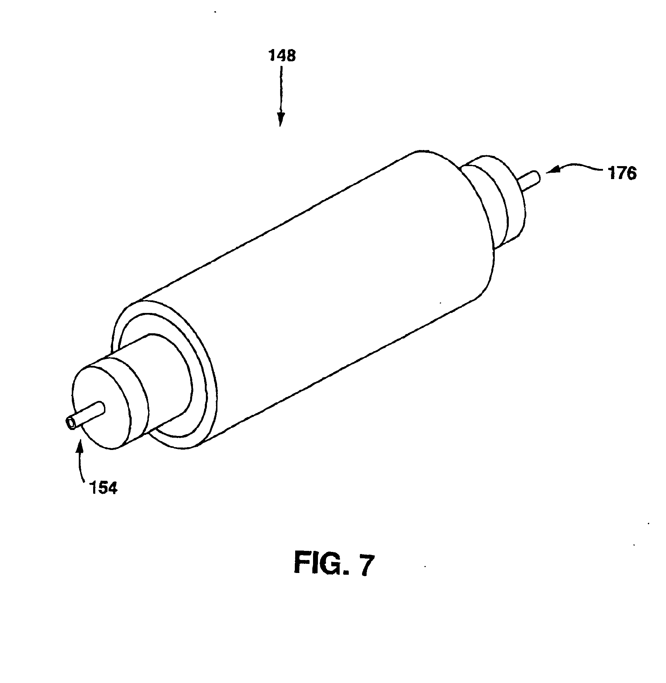

FIGS 7-11 show a three coil embodiment of the solenoid pump. The permanent magnet solenoid pump 148 comprises a pump cylinder 156; a pump piston 158, a biasing element 160, an inlet valve 162, an outlet valve 164, a permanent magnet 166, a first coil 168, and a second coil 170. The solenoid pump 148 is coupled to the electronics 146, the therapeutic substance reservoir outlet 152, and the needle 154. The solenoid pump 148 is configured for pumping therapeutic substance 136 from the reservoir 142 through needle 154 at a programmed rate.

The pump cylinder 156 has an inlet enclosure 172, an outlet enclosure 174, a therapeutic substance inlet 176, and an infusion needle 154. The inlet enclosure 172 transitions the pump cylinder 156 to the therapeutic substance inlet 176. The outlet enclosure 174 transitions the pump cylinder 156 to the infusion needle 154. The therapeutic substance inlet 176 is coupled to a therapeutic substance reservoir outlet 152 and coupled to the inlet enclosure 172 on the pump cylinder 156. Some embodiments can include a piston seal 178 positioned between the pump cylinder 156 and the pump piston 158 to reduce therapeutic substance 136 flow between the pump piston 158 and the pump cylinder 156 and provide other functions. The piston seal 178 can be configured to serve as a guide for the biasing element 160 and to cushion the pump piston 158 at the end of pump piston 158 retraction for the intake stroke. The piston seal 178 is manufactured from a resilient material with good sealing qualities such as synthetic rubber, PTFE, silicone, and the like.

The pump piston 158 is moveable within the pump cylinder 156 and has a piston inlet end 180, a piston outlet end 182, and a piston fluid path 184. The pump piston 158 forms an inlet chamber 186 between the pump piston 158 and the inlet enclosure 172 and a pumping chamber 188 between the pump piston 158 and the outlet enclosure 174. The inlet chamber 186 contains the therapeutic substance 136 that is displaced when the pump piston 158 retracts. The pumping chamber 188 contains the therapeutic substance 136 that is displaced when the pump piston 158 is actuated. The piston fluid path 184 is configured to provide fluid communication between the inlet chamber 186 and the pumping chamber 188 that is controlled by the inlet valve -162. The piston fluid path 184 can take a wide variety of forms such as a central fluid path, a side fluid path, a partial central and partial side fluid path, and the like.

The biasing element 160 is positioned in the pump cylinder inlet chamber 186 between the pump piston 158 and the inlet enclosure 172. The biasing element 160 exerts force on the pump piston 158 to expulse therapeutic substance 136 through the infusion needle 154. In some embodiments, the biasing element 160 exerts substantially the sole force on the pump piston 158 to expulse therapeutic substance 136 through the infusion needle 154. The biasing element 160 also provides force to maintain the pump piston 158 in an actuated position until retracted to seal the inlet valve 162 against the outlet enclosure 174 to provide redundant protection against unintended flow of therapeutic substance 136 to the patient. The biasing element 160 can be one or more of a wide variety of biasing structures that are selected to provide the desired biasing force on the pump piston 158. The desired force of the biasing element 160 in a particular embodiment is the force required to overcome any frictional losses during the pump piston 158 expulsion stroke and to generate pressure to open the outlet valve 164. Some specific embodiments of the biasing element 160 include a spring, a coil spring, and the like.

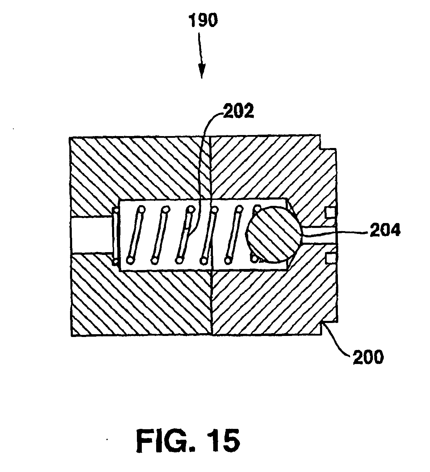

The inlet valve 162 is carried on the pump piston outlet end 182. The inlet valve 162 can be a variety of inlet valves 162 such as a flapper valve, annular flapper valve, ball valve, reed valve, duckbill valve, poppet valve, and the like. The outlet valve 164 is carried in the outlet enclosure 174 and coupled to the infusion needle 154. The outlet valve 164 improves solenoid pump 148 safety by substantially preventing unintended flow of therapeutic substance 136 when the reservoir 142 pressure is greater than the infusion site 134 pressure. The outlet valve 164 improves solenoid pump 148 accuracy by maintaining sufficient back pressure to keep the inlet valve 162 closed during therapeutic substance 136 expulsion through the infusion needle 154 so that addition therapeutic substance 136 is not infused when the reservoir 142 pressure is greater than the infusion site 134 pressure. The outlet valve 164 can be a variety of outlet valves 164 such as a flapper valve, ball valve, reed valve, duckbill valve, poppet valve, and the like. An outlet valve embodiment is shown in FIG. 15.

Some embodiments of the solenoid pump 148 can include an anti-cavitation valve 190 positioned in fluid communication with the therapeutic substance inlet 176. The anti-cavitation valve 190 substantially prevents therapeutic substance 136 in the inlet chamber 186 from flowing back through the therapeutic substance inlet 176 during pump piston 158 retraction. Since the therapeutic substance 136 cannot flow backwards, pressure in the inlet chamber 186 increases as the pump piston 158 retracts causing the therapeutic substance 136 to flow through the piston fluid path 184 without causing the pump chamber 188 pressure to drop low enough to cause dissolved gasses to come out of solution. Also by substantially preventing the back flow of therapeutic substance 136 through the therapeutic substance inlet 176 during pump piston 158 retraction, piston pump 158 efficiency is improved because wasted therapeutic substance 136 flow is minimized. The anti-cavitation valve 190 can be a wide variety of anti-cavitation valves 190 such as a flapper valve, annual flapper valve, ball valve, reed valve, duckbill valve, poppet valve, and the like. In addition to displacing therapeutic substance 136 that operates the inlet valve 162, outlet valve 164, and anti-cavitation valve 190, the pump piston 158 carries a permanent magnet 166.

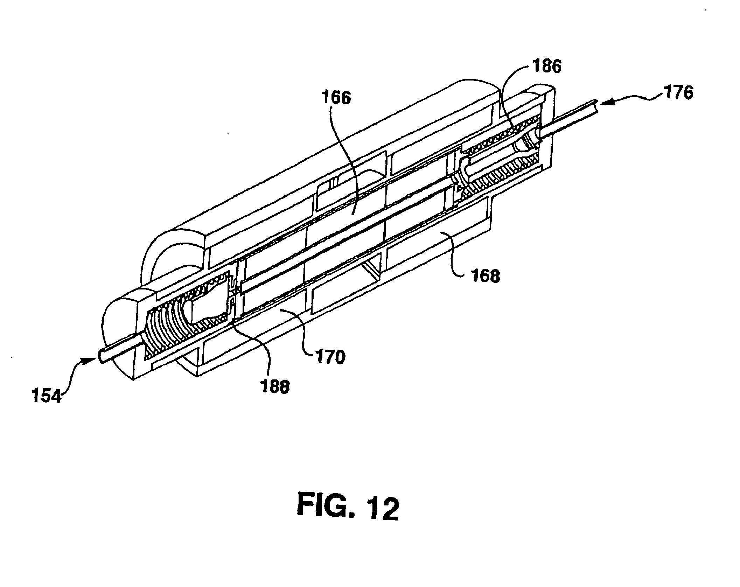

The permanent magnet 166 is at least a first permanent magnet 166 having a first pole 192 and a second pole 194. The permanent magnet 166 is carried by the pump piston 158 and acted on by the magnetic fields created by the coils 167 that include at least the first coil 168 and at least the second coil 170. When the first coil 168 and second coil 170 are energized, the coils 167 produce an electromagnetic axial force that acts on the permanent magnet 166 to impart motion to the pump piston 158. In some embodiments, there can be more than one permanent magnet 166 such as a first permanent magnet 166, a second permanent magnet 196, a third permanent magnet 198 and so forth. Also in some embodiments, there can be more than a first coil 168 and second coil 170 such as a third coil 171 and so fourth. When using more than one permanent magnet 166, like poles are positioned adjacent to one another, and there are $N$-1 operational permanent magnets 166 where N is the number of coils 167 in the range from about 3 to 10. The permanent magnet 166 is manufactured from a hard ferromagnetic material such as samarium cobalt, neodymium, ceramic, alnico, and the like. Since the permanent magnet 166 material is typically not therapeutic substance compatible or biocompatible, the permanent magnet 166 is typically isolated from the therapeutic substance 136 by the piston fluid path 184 and the pump piston 158 sealed is by the piston inlet end 180 and piston outlet end 182. Positioned in operating relationship to the permanent magnet 166 are the first coil 168 and the second coil 170. An alternative solenoid pump 148 embodiment using two coils 167 is shown in FIGS. 12-13.

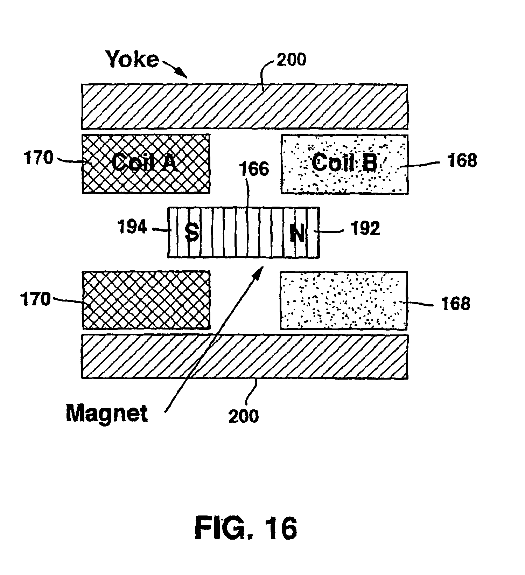

FIGS. 16-19 show block diagram of some of the permanent magnet solenoid pump 148 permanent magnet 166 and coil 167 embodiments. There is at least a first coil 168 configured around the pump cylinder 156 positioned adjacent to the first pole 192 of the permanent magnet 166, and at least a second coil 170 configured around the pump cylinder 156 positioned adjacent to the second pole 194 of the permanent magnet 166. The first coil 168 and the second coil 170 can be reversed in relation to the permanent magnet 166, so the first coil 168 is adjacent to the second pole 194 and the second coil 170 is adjacent to the first pole 192. Other embodiments of the permanent magnet solenoid pump 148 can have more than a first coil 168 and second coil 170 such as a third coil 171 or N operational coils where N is an integer in the range from about 3 to 10. When the solenoid pump 148 is configured with $N$ coils there will be $N$-1 operational permanent magnets 166. The following discussion will use the term coil 167 to refer a first coil 168 and a second coil 170, or $N$ coils and permanent magnet 166 to refer to a first permanent magnet 166 or $N$-1 permanent magnets.

The coils 167 are typically wound in opposite directions, so current flows in opposite directions to generate opposing magnetic fields. Alternatively, the coils 167 can be wound in the same direction and the voltage polarity applied to the coils 167 can be opposing to cause current to flow in opposite directions to generate opposing magnetic fields. The coils 167 are configured to create a force in one direction for one permanent magnet pole 192 and in the same direction for the other permanent magnet pole 194. The coils 167 are energized for pump piston 158 retraction toward the inlet enclosure 172 to fill the pump chamber 188 from the inlet chamber 186. Since the coils 167 are not typically compatible with therapeutic substances or biocompatible, the coils 167 are typically isolated from the therapeutic substance 136 by the pump cylinder 156 and isolated from the patient 138 by the housing 141. The pump cylinder 156 is manufactured from any therapeutic substance compatible material that meets design requirements such as titanium, tantalum, stainless steel, plastic, ceramic, and the like. The permanent magnet 166 and coils 167 can be inserted into the pump cylinder 156 along with the pump piston 158 and then the inlet enclosure 172 and outlet enclosures 174 can be welded to isolate the permanent magnet 166 and coils 167 from the therapeutic substance 136. The coil's 167 electromagnetic axial force can be enhanced with a magnetically permeable yoke 200 and pole pieces 202.

The magnetically permeable yoke 200 and pole pieces 202, although not required for operation, enhance the electromagnetic axial force created by the coils 167. The magnetically permeable yoke 200 is placed around the coils

EP 1 385 570 B1

167 to improve magnetic efficiency. The magnetically permeable yoke 200 also shields the permanent magnet 166 from external high magnetic fields, until the magnetically permeable yoke 200 becomes saturated, to protect the permanent magnet 166 from demagnetization. The external high magnetic field can be created by equipment such as Magnetic Resonance Imaging (MRI) equipment, magnetic security equipment, and the like. The pole pieces 202 can be positioned adjacent to one or more of the permanent magnet's 166 poles. For example, a first pole piece 204 can be carried on the pump piston 158 between the permanent magnet 166 and the inlet enclosure 172 to improve magnetic coupling. A second pole piece 206 can be carried on the pump piston 158 between the permanent magnet 166 and the outlet enclosure 174 to improve magnetic coupling.

When the pump piston 158 is fully positioned toward the inlet enclosure 172, the maximum pump chamber 188 volume is created. The pump chamber 188 has a pump chamber 188 volume comprising a stroke volume and a dead volume. The stroke volume is in the range from about 0.5 micro liters to about 5.0 micro liters. The sum of an inlet valve 162 opening pressure and the outlet valve 164 opening pressure exceeds the maximum pressure of the reservoir 142 less the infusion site 134 pressure to substantially prevent free flow of therapeutic substance 136 to the patient. The dead volume is less than half the stroke volume in the range from about 0.25 micro liters to about 2.5 micro liters. The solenoid pump's 148 small dead volume compared to the stroke volume improves the solenoid pump's 148 capability to pass air because of the low vacuum pressure that is typically generated in the pump chamber 188. The inlet valve 162 and outlet valve 164 opening pressures are selected to prevent unintended infusion under extreme operating conditions. Unintended infusion is substantially prevented by selecting the inlet valve 162 opening pressure and the outlet valve 164 opening pressure so the sum of these pressures is greater than the maximum pressure difference between the reservoir 142 and the infusion site 134. For example, unintended infusion is prevented when the reservoir 142 pressure is high and the ambient pressure (typically the same as the infusion site 134 pressure) is low that can occur when the reservoir 142 is full and the patient is exposed to high temperature at high altitude.

The solenoid pump's 148 ability to pass air and operate accurately is a function of the solenoid pump's 148 compression ratio, the reservoir outlet 152 pressure, the infusion needle 154 pressure, and outlet valve 164 cracking (opening) pressure. For adiabatic systems with ideal gases, the compression ratio in the pump chamber 188 can be expressed as $CR_{pc} = \dfrac{V_{pc\ final}}{V_{pc\ initial}}$ (Equation 1) where $CR_{pc}$ is the compression ratio in the pump chamber 188, $V_{pc\ final}$ is the final volume in the pump chamber 188 calculated by *(strokevolume + pump chamber dead volume)* where *stroke volume= piston area x piston stroke*, and $V_{pc\ initial}$ is the initial volume in the pump chamber 188 also known as the *pumpchamberdead volume* which is also the pump chamber volume remaining after the pump piston 158 has expulsed the stroke volume. The compression ratio in the inlet chamber 186 can be expressed as $CR_{ic} = \dfrac{V_{ic\ final}}{V_{ic\ initial}}$ (Equation 2) where $CR_{ic}$ is the compression ratio in the inlet chamber 186 and $V_{ic}$ is the volume in the inlet chamber 186, $V_{ic\ final}$ = *(stroke volume+inletchamberdead volume),* and $V_{ic\ initial}$ = *(inlet chamber dead volume).* From these relationships, it is apparent that pump chamber 188 pressure will be decreasing and inlet chamber 186 pressure will be increasing as the pump piston 158 retracts. For therapeutic substance 136 to flow into the pump chamber 188 when gas bubbles are present, the pressure in the pump chamber 188 during the pump piston 158 stroke must drop substantially below the inlet chamber 186 pressure. For therapeutic substance 136 to flow out of the pump chamber 188, the expulsion pressure must be greater than the infusion needle 154 pressure. Therapeutic substance 136 flows through the outlet valve 164 when $P_{pc} \geq P_a + P_{ovc}$, where $P_{pc}$ is the pressure in the pump chamber 188, $P_a$ is the ambient pressure at the infusion outlet 154, and $P_{ovc}$ is outlet valve 164 cracking (opening) pressure. By selecting the appropriate outlet valve 164 cracking pressure the risk of unintentional infusion can be substantially eliminated.

The permanent magnet solenoid pump 148 is designed to be energy efficient consuming less than about 3.0 Joules per milliliter (J/ml) at about 137,895 pascals (20 pounds/square inch) back pressure. The solenoid pump 148 is about 5% to 10% efficient in terms of electrical energy input and fluid work output. The energy efficiency of the solenoid pump 148 to a large degree determines energy efficiency of the therapeutic substance delivery patch 130 and the size of power source 144 needed. For example, doubling solenoid pump 148 efficiency can theoretically result in nearly a 50% reduction in energy consumption and a 50% reduction in the size of power source 144. Additionally, the solenoid pump 148 can be a wide variety of sizes. The design of the solenoid pump 148 permits small sized configuration such as less than about 20.0 mm long by about 6.0 mm in diameter with a total volume less than about 0.5 cc. The small size of the solenoid pump 148 also permit having a small therapeutic substance delivery patch 130 that can be more inconspicuously attached to a patient's skin such as under clothes, including on arms, legs, torso or other areas.

**Operation (Patch 130 Embodiment)**

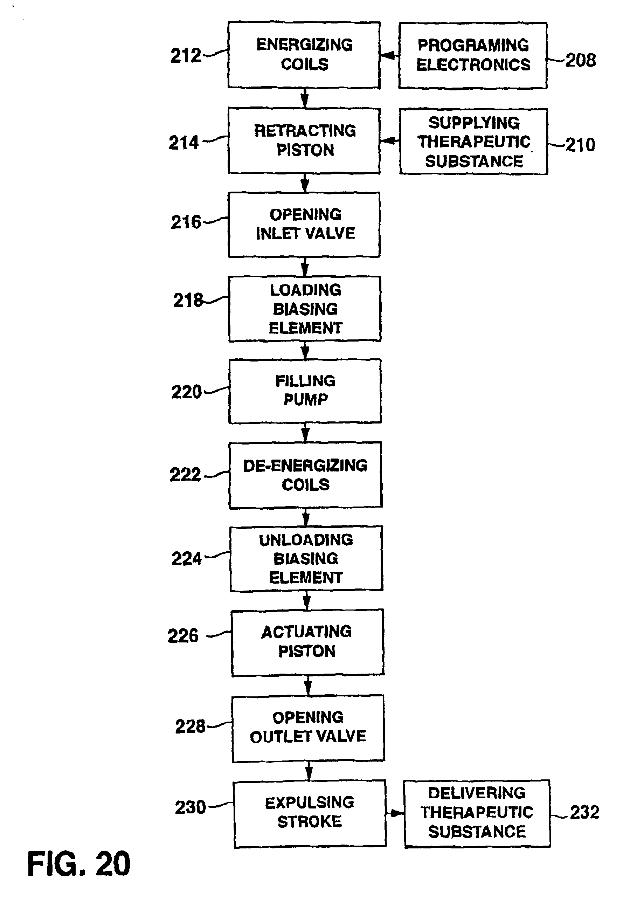

FIG. 20 shows a flowchart of a method for operating a therapeutic substance delivery patch 130 having a permanent magnet solenoid pump 148 embodiment. The therapeutic substance 136 is supplied from a therapeutic substance reservoir 142 to a therapeutic substance inlet 176. The solenoid pump 148 generally operates by retracting a pump piston 158 and then actuating the pump piston 158 while operating valves to deliver therapeutic substance 136 through an infusion needle 154 at a programmed rate. This operation is repeated a predetermined number of times at predetermined intervals to delivery therapeutic substance 136 at the programmed rate. For example, a solenoid pump 148 with a stroke volume of 2.0 micro liters would typically be operated from a maximum of about 10 cycles per second (Hz) to achieve an infusion rate of 1.2 milliliters per minute to a minimum of about 1.0 cycle per hour to achieve an infusion rate of about 48 micro liters per day.

Retracting the pump piston 158 is initiated when a first coil 68 for current flow in a first direction is energized 112 and a second coil 170 for current flow in an opposite direction are energized 212 to create a electromagnetic axial force. The pump piston 158 is retracted 214 when the electromagnetic axial forces acts on a permanent magnet 166 carried on the pump piston 158. While the pump piston 158 is retracting, an inlet valve 162 is opened 216 and a biasing element 160 is loaded. A pump chamber 188 is filled with therapeutic substance 136 through the inlet valve 162 while the pump piston 158 is being retracted 214. In an embodiment having a piston seal 178, during pump piston 158 retraction the piston seal 178 can also be configured to dampen the shock when the pump piston 158 reaches its fully retracted position. By dampening this shock, the piston seal 178 can reduce some wear and noise that occurs when the pump piston 158 reaches its fully retracted position. In embodiments having an anti-cavitation valve 190, the anti-cavitation valve 190 prevents therapeutic substance 136 in the inlet chamber 186 from flowing back to the therapeutic substance reservoir 142 when the pump piston 158 retracts. The anti-cavitation valve 190 helps maintain higher pump chamber 188 pressures during pump piston 158 retraction, which makes it easier to pass air bubbles.

During pump piston 158 retraction assuming there is anti-cavitation valve 190 and both the inlet chamber 186 and pump chamber 188 are filled with therapeutic substance 136, the pressure in the inlet chamber 186 will increase rapidly due to the incompressibility of liquids which will cause the therapeutic substance 136 to flow through the piston fluid path 184 into the pump chamber 188 without causing the pump chamber 188 pressure to decrease to the level that would cause gasses to come out of solution. After the pump piston 158 is retracted, operation of the solenoid pump 148 continues when the pump piston 158 is actuated.

Actuating the pump piston is initiated when the first coil 168 for current flow in the first direction is de-energized 222, and the second coil 170 for current flow in the opposite direction is de-energized 222 to collapse the electromagnetic axial force. As the electromagnetic axial force collapses, the biasing element 160 is unloaded, and the pump piston 158 is actuated by the biasing element 160 driving the pump piston 158 toward the outlet enclosure 174. While the pump piston 158 is being actuated 226, pressure generated in the pumping chamber 188 opens the outlet valve 164. The open outlet valve 164 permits a stroke volume to be expulsed through the infusion needle 154 while the pump piston 158 is actuated 226. The therapeutic substance 136 discharged through the infusion needle 154 is delivered at a programmed rate. In some embodiments during pump piston actuation 226, the piston seal 178 substantially prevents therapeutic substance 136 from flowing around the pump piston 158 back into the inlet chamber 186. The previously discussed method embodiment elements are presented in a general sequence that is intended to be limiting only when a particular element is required to be sequence in a certain way for the entire method embodiment to be practical. Pump piston actuation 226 can be mathematically characterized.

During piston actuation 226 to expulse therapeutic substance 136, when the pump chamber 188 volume is decreasing and the inlet chamber 186 volume is increasing the following relationships exist. The final pressure in the pump chamber 188 can be express as $P_{pc\ final} = \dfrac{P_{pc\ initial}}{CR_{pc}}$ (Equation 3) where $P_{pc\ final}$ is the final pressure in the pump chamber 188, $P_{pc\ initial}$ is the initial pressure in the pump chamber 188, and $CR_{pc}$ is the compression ratio in the pump chamber 188. The final pressure in the inlet chamber 186 can be expressed as $P_{ic\ final} = \dfrac{P_{ic\ initial}}{CR_{ic}}$ (Equation 4) where $P_{ic\ final}$ is the final pressure in the inlet chamber 186, $P_{ic\ initial}$ is the initial pressure in the inlet chamber 186, and $CR_{ic}$ is the compression ratio in the pump chamber 188. Therapeutic substance 136 flows through the outlet valve 164 when $P_{pc\ final} \geq P_a + P_{ovc}$ (Equation 5) where $P_{ic}$ is the initial pressure in the inlet chamber 186, $P_a$ is the ambient pressure at the pump outlet, and $P_{ovc}$ is outlet valve 164 cracking (opening) pressure. The above relationships assume that there is an anti-cavitation valve 190, there is no air in the pump chamber 188, and since liquids are essential incompressible $P_{ic}$ decreases as the pump piston 158 is actuated.

Thus, embodiments of the solenoid pump 148 are disclosed to increase energy efficiency, increase accuracy, reduce the residential space requirement, improve therapeutic substance compatibility, and provide many other improvements. One skilled in the art will appreciate that the present invention can be practiced with embodiments other than those disclosed. The disclosed embodiments are presented for purposes of illustration and not limitation, and the present invention is limited only by the claims that follow.

**Detailed System Example (IMD 312 and Patch 320)**

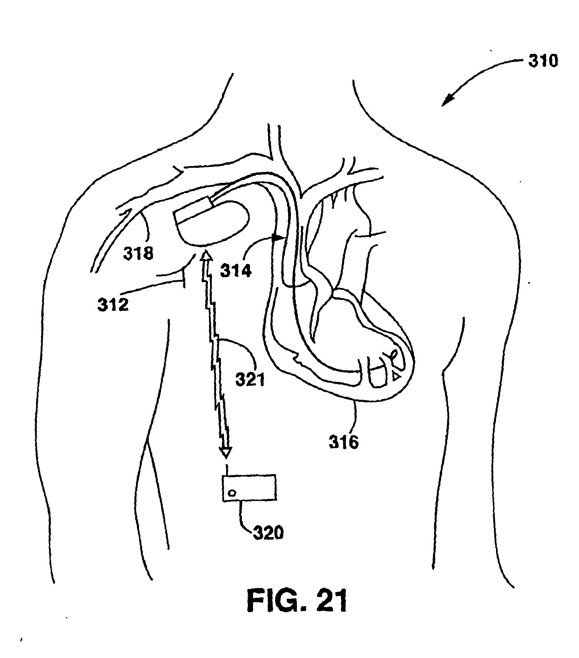

A detailed system example different from the examples provided above including an implanted medical device 30 and therapeutic substance delivery patch 32 utilized in conjunction with delivery of therapy by the implanted medical device 30 is provided in conjunction with figures 21-27.

FIG. 21 is an illustration of an implantable medical device, IMD 30, adapted for use to communicate with an externally mounted therapeutic substance delivery patch 32. The IMD implanted in patient 10 includes IMD 312. IMD 312 is a pacemaker, implanted cardioverter/defribillator (ICD) or combined pacemaker/ICD device. In accordance with conventional practice in the art, IMD 312 is housed within a hermetically sealed biologically inert outer casing which may itself be conductive so as to serve as an indifferent electrode in the IMD's pacing cardioversion/sensing circuit. One or more pacemaker leads collectively identified with reference number 314 are electrically coupled to IMD 312 in a conventional manner and extend into the patient's heart 316 via vein 318. Disposed generally near the distal end of leads 314 are one or more exposed conductive electrodes for receiving electrical cardiac signals and/or for delivering electrical pacing and/or cardioversion/defibrillation stimuli to heart 316. As will be appreciated by those of ordinary skill in the art, leads 314 may be implanted with distal ends situated in the atrium and/or ventricle of heart 316.

In this embodiment of the invention, patch 32 is patch 320. As depicted in FIG. 21, patch 320 is linked via telemetry transmission 321 to IMD 312.

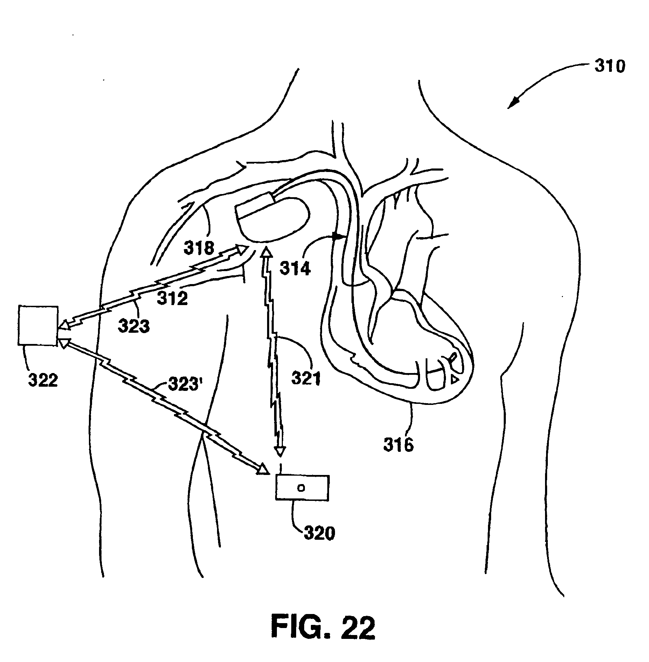

FIG. 22 is a variation of FIG. 21 in which programmer 322 is shown in communication with implanted medical device 312 and patch 320. Specifically, programming unit 322 is in telemetry or wireless communication with implanted medical device 312 and patch 320 via uplink and downlink communication channels 323 and 323', respectively. Programmer 322 described herein with reference to FIG. 21 is disclosed in more detail in U.S. Patent No. 5,345,362 issued to Thomas J. Winkler entitled "Portable Computer Apparatus with Articulating Display Panel"

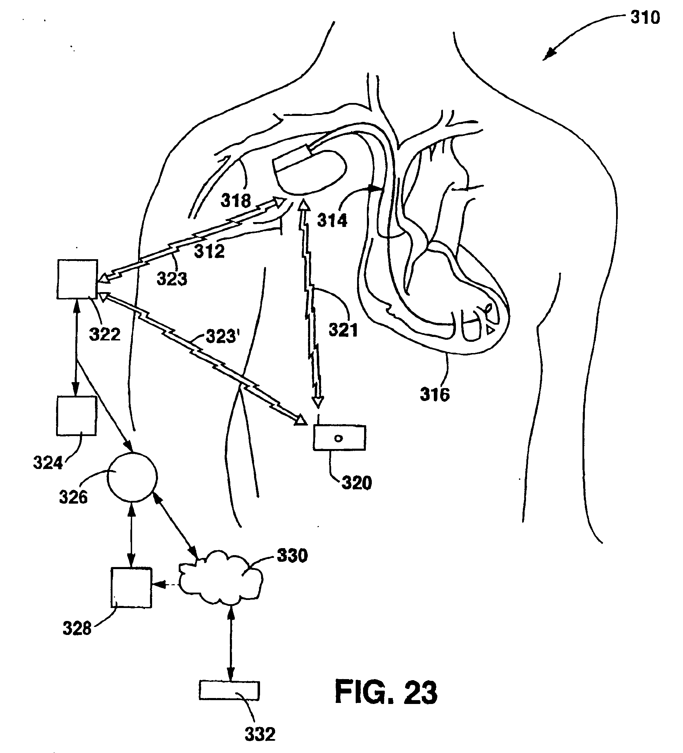

FIG. 23 is another embodiment of the present invention wherein data is communicated using various media to transfer information from IMD 312 and patch 320 via programmer, IRM or equivalent device 322. As discussed hereinabove data from IMD 312 and patch 320 is uplinked to device 322 from which it may be directed to a PC 324 or server 326 using, for example, a modem, an ISDN line, fiber optic, cable, infrared, bluetooth-enabled or equivalent direct or wireless communication systems. Server 326 is also accessible directly to qualified users at station 328. Further, server 326 may be accessible via Internet 330 to remote users 332.

With this exemplary communication network, caregivers, physicians and other qualified personnel may be able to access and review or reprogram the operations of IMD 312 and patch 320 remotely. For example, user 328 may use a LAN1 or other secure lines to access server 326 from which either current or stored data relating to the operation of IMD 312 and patch 320 could be obtained for evaluation and adjustment, or remote patient monitoring. Similarly, remote users at station 332 may be able to access operational and functional data of IMD 312 and patch 320 via Internet 330.

One of the aspects of this embodiment of the present invention is the use of a transdermally operable patch 320. It is similar to the electrotransport drug delivery system disclosed in Design Patent No. 384,745 to Lattin et al. Further, a similar device is disclosed in U.S. Patent No. 5,995,869 to Cormier et al. Additionally, electrotransport delivery device with voltage boosting circuit is disclosed in U.S. Patent No. 6,035,234 to Riddle et al. And an electrotransport device and method of setting the output using the same drug delivery device is disclosed in U.S. Patent No. 6,086,572 to Johnson et al.

The present invention implements a highly adaptable communication link between the implanted device and the drug delivery device disclosed in the prior art. The communication system as indicated hereinabove without limitation could be telemetry or as substantially described in U.S. Patent No. 5,683,432 and 5,843,139, or an equivalent medical device communication system such as the one disclosed in U.S. Patent No. 4,987,897 to Funke. Accordingly, the present invention provides a drug delivery system as defined in the claims.

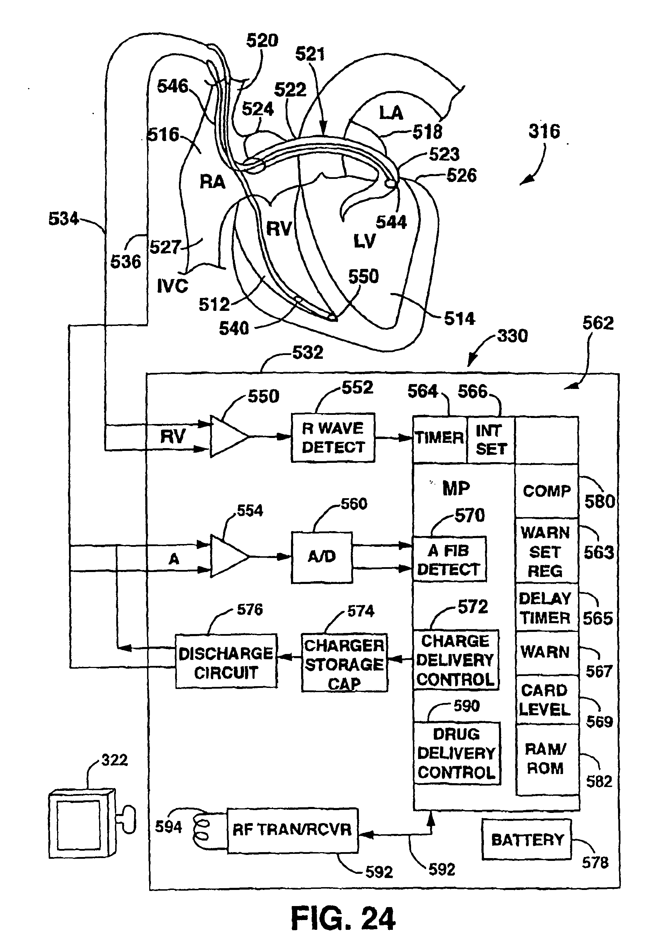

Referring now to figure 24, a fully implantable atrial cardioverter system 312 embodying the present embodiment of the invention in association with a schematically illustrated human heart 316 in need of atrial fibrillation monitoring and potential cardioversion of the atria 516, 518 and an external programmer 322 are shown. The atrial cardioverter system 312 is capable of the sequential initiation of delivery of a pain alleviating analgesic at therapeutic levels followed by delivery of atrial cardioversion electrical energy pulses or shocks of sufficient amplitude and duration to effectively cardiovert the heart 316 in atrial fibrillation. The portions of the heart 316 illustrated in figure 23 are the right ventricle (RV) 512, the left ventricle 514, the right atrium (RA) 516, the left atrium 518, the superior vena cava (SVC) 520, the CS 521 including the coronary sinus (CS) ostium or opening 524, the left ventricular free wall 526 and the inferior vena cava 527.

The system 330 generally includes an enclosure 532, for hermetically sealing the internal circuit elements, battery, telemetry antenna, a bipolar RV lead 534, and a RA-CS lead 536. The enclosure 532 and leads 534 and 536 are arranged to be implanted beneath the skin of a patient so as to render the atrial cardioverter system 330 fully implantable.

The RV lead 534 preferably comprises an endocardial bipolar lead having electrodes 538 and 540 arranged for establishing electrical contact with the right ventricle 512 of the heart 316. The electrodes 538 and 540 permit bipolar sensing of ventricular depolarizations or R-waves in the right ventricle 512. As illustrated, the lead 534 is preferably fed through the SVC 520, the right atrium 516, and then into the right ventricle 512 to lodge the electrodes 538, 540 in the apex thereof as illustrated.

The RA-CS lead 536 generally includes a tip or CS cardioverting electrode 544 and a proximal ring or RA cardioverting electrode 546 as shown in US patent No. 5,165,403, for example. As illustrated, the RA-CS lead 536 is flexible and arranged to be passed down the superior vena cava 520, into the right atrium 516, into the coronary sinus ostium 524. The CS electrode 544 is advanced into the coronary sinus channel 521 of the heart near the left side thereof so that the first or tip electrode 544 is within the coronary sinus channel 521 either within the coronary sinus 522 adjacent the left ventricle 514 and beneath the left atrium 518 or most preferably within the great cardiac vein 523 adjacent the left ventricle 514 and beneath the left atrium 518. The electrodes 544 and 546 are spaced apart such that when the CS electrode 544 is positioned as described above, the RA electrode 546 is in the right atrium 516. The CS electrode 544 together with the RA electrode 546 provides bipolar sensing of heart activity in the atria 516 and 518.

The CS electrode 544 and the RA electrode 546 also provide for the delivery of defibrillating electrical energy to the atria. Because the CS electrode 544 is located beneath the left atrium 518 near the left ventricle 514 and the RA electrode 546 is within the right atrium 516, the cardioverting electrical energy, when applied between these electrodes will be substantially confined to the atria 516 and 518 of the heart 316. As a result, the electrical energy applied to the right ventricle 512 and left ventricle 514 when the atria are cardioverted or defibrillated will be minimized. This greatly reduces the potential for ventricular fibrillation of the heart to be induced as a result of the application of cardioversion electrical energy of the atria of the heart.

Further electrode systems and cardioversion pathways have been disclosed and are suitable for use in the practice of the present invention. One such atrial cardioversion electrode system is disclosed in the article "Safety and Feasibility of Transvenous Cardioversion in Atrial Tachycardia", by Blanc et al., published in Cardiac Pacing, edited by Gomez, Future Pub. Co., 1985, pp 1526-1529. This electrode system employs a single lead with electrodes located in the right atrium and in the pulmonary artery. Delivery of atrial cardioversion shocks between an RV electrode and a subcutaneous electrode is disclosed in US Patent No. 5,292,338. Delivery of atrial defibrillation pulses between a coronary sinus electrode and a subcutaneous electrode is also disclosed in US Patent No. 5,314,430.

A further suitable atrial cardioversion electrode system is disclosed in US Patent No. 5,549,642. The electrode system disclosed therein includes an RA/SVC electrode (alone or optionally coupled to a subcutaneous electrode) and a CS electrode. The elongated RA/SVC electrode appears to provide atrial defibrillation thresholds in the range of about 1.0 Joule or less across a substantial portion of the patient population which represents a substantial improvement over the RA or SVC to CS/great vein electrode system employed in the above-referenced '403 patent.

Any of the above atrial cardioversion electrode systems and associated atrial and/or ventricular leads may be used in the practice of the present invention. However, even an approximately 1.0 joule cardioversion shock can be painful to a substantial portion of the population, particularly since atrial fibrillation episodes repeat frequently, requiring frequent cardioversion.

Within the enclosure 532, the system 330 includes a ventricular sense amplifier 550 coupled to the RV lead 534 to receive electrical signals in the ventricle across the bipolar electrode pair 538, 540 and an R-wave detector 552 to detect the R-waves therefrom. The ventricular sense amplifier 550 and the R-wave detector 552 form a first detecting means that senses R-waves in the electrogram transmitted to ventricular sense amplifier by the RV lead 534. The R-wave detector 552 is of the type well known in the art, which provides an output pulse upon the occurrence of an R-wave being sensed during a cardiac cycle of the heart. The delivery of the atrial defibrillation shock or pulse is timed from the R-wave employing the ventricular timer 564 as described below.

The lead and electrode systems in certain embodiments of the above-referenced '338, '403 and '430 and '642 patents include an RV defibrillation electrode positioned on an RV lead inserted into the right ventricle and a pair of ventricular sense electrodes. Alternatively, in the atrial cardioversion system depicted in figure 24, common ventricular pacing leads having bipolar screw-in ventricular electrodes of this type may be employed as pace/sense electrodes 538, 540.

An atrial sense amplifier 554 is coupled to the RA-CS lead 536 to receive electrical signals or P-waves across the right atrium 516. The atrial sense amplifier 554 forms a second detecting means for detecting P-wave atrial activity of the heart picked up by the CS electrode 544 and RA electrode 546 of the RA-CS lead 536. The P-wave output signal of the atrial sense amplifier 554 is coupled to an analog to digital converter 560 which converts the analog signal representative of the atrial activity of the heart to digital samples for further processing to determine if atrial fibrillation is present and if the atrial cardioversion shock is effective in converting the atria to a normal atrial rate.

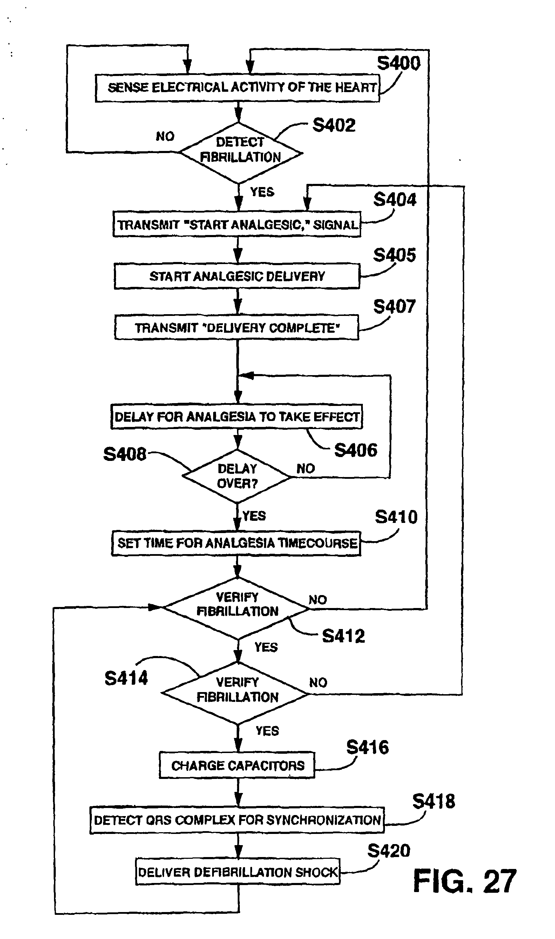

The enclosure 532 of the atrial cardioverter system 330 further include a microcomputer 562 that is preferably implemented in a manner disclosed in the above-referenced '338 patent and further as described hereinafter with respect to the flow diagram of figure 27. The implementation of the microcomputer 562 in accordance with this embodiment of the present invention results in a plurality of functional stages and RAM/ROM 582 for storing operating algorithms and programmable parameters as well as accumulated operating data for subsequent telemetry out to the external programmer 322.

The circuitry includes the ventricular timer 564 for timing various intervals that recur in each QRST cycle as well as the R-wave synchronization time interval, an interval set stage 566 for selecting time intervals to be timed out in the ventricular timer 564, a delay timer 565 for timing out further delay times set in interval set stage 566 for the delivery of the pain alleviating therapy, an optional patient warning device 567 and warning set register 563, a cardioversion energy level set stage 569, an atrial fibrillation detector 570, a charge delivery control stage 572, an analgesic delivery control stage 590, and a computation stage 580.

The microcomputer 562 is arranged to operate in conjunction with RAM/ROM memory 582 which may be coupled to the microcomputer 562 by a multi-bit address bus and a bi-directional multiple-bit data bus. This permits the microcomputer 562 to address desired memory locations within the memory for executing write or read operations. During a write operation, the microcomputer 562 stores data, such as time intervals or operating parameters in the memory 582 at the addresses defined by multiple-bit data bus. During a read operation, the microcomputer 562 obtains data from the memory at the storage locations identified by the multiple-bit addresses provided over the address bus and receives the data from the memory 582 over the bi-directional data bus. Data related to the detections of atrial fibrillation and the deliveries of the therapies may be recorded in the RAM memory 582 for interrogation and telemetry out to the external programmer 322 in a manner well known in the art.

Detection of atrial fibrillation may be accomplished in atrial fibrillation detector 570, in conjunction with computation stage 580, of microcomputer 562 from the digitized P-waves detected by atrial sense amplifier 554 using any of the various atrial fibrillation detection methodologies known to the art. Generally, atrial fibrillation may be detected in response to an extended series of high rate (e.g. 240 BPM or greater) atrial depolarizations or P-waves. If greater specificity for atrial fibrillation is desired, analysis of regularity of rate waveform morphology may also be employed.

Termination of atrial fibrillation may be detected in response to a decrease in the rate of atrial depolarizations and/or an increase in their regularity.

Appropriate detection methodologies are disclosed in the article "Automatic Tachycardia Recognition", by Arzbaecher et al., published in PACE, Vol. 7, May-June 1984, part II, pages 541-547 and in PCT Application No. US92/02829, Publication No. WO 92/18198 by Adams et al. In the PCT application, careful synchronization of the high voltage atrial defibrillation pulse to the ventricles to avoid induction of ventricular tachycardia or fibrillation is also discussed.

In addition, in the context of devices which automatically detect the occurrence of atrial fibrillation, the patient may optionally be warned of the detection of atrial fibrillation to be ready for the delivery of the atrial cardioversion shock through operation of the warning device 567. In this alternate variation of the embodiment of the invention, a warning may be provided to the patient of the diagnosis of atrial fibrillation and the commencement of delivery of the pain alleviation drug therapy. The warning may be effected in the manner described in US Patent No. 5, 332,400, but is preferably effected by energizing a piezoelectric crystal oscillator that oscillates at an audible frequency intense enough for the patient to hear it and take precautions, if necessary. The patient may also optionally be provided with a limited function programmer 322 for use in communicating a command to the microcomputer 562 to prevent delivery of the cardioversion shock until the patient feels the effects of the pain alleviation therapy, at which time the patient may employ the programmer 322 to enable delivery of the cardioversion shock, subject to re-verification of the presence of the atrial fibrillation.

In this regard, the system 330 also includes the warning set register 563, the delay timer 565, and the warning device 567 that are utilized for generating the warning alarm for the patient when the atrial fibrillation detector 570 determines that the atria are in fibrillation. The warning device 567 may constitute an audible alarm sounding piezoelectric crystal oscillator for warning the patient that atrial fibrillation has been detected and that cardioverting electrical energy will be applied to the patient's atria.

If a programmer 322 is provided, it may also optionally include a patient activated command signal to initiate the delivery of the pain alleviating and cardioversion therapies in response to symptomatic atrial fibrillation. In this context as well, the ability to use the programmer 322 to delay the delivery of the cardioversion pulse until the patient has felt the effects of the pain alleviating therapy is believed valuable.

After the fibrillation detection warning is delivered to the patient, or after the patient requests cardioversion therapy by means of the programmer 322, register 563 is set to indicate that the patient has received the fibrillation detection warning or has requested therapy. Immediately thereafter, the delay timer 565 starts timing the warning delay period and initiates communication to drug delivery system 320 via RF transmitter 592 and antenna 594. The delay period defines a time interval from when the patient receives the warning or requests therapy to when the patient should first expect to receive the cardioverting electrical energy. The delay time is preferably programmable between one minute and twenty minutes to afford sufficient time to permit the pain alleviating therapy to take effect and for the patient to prepare for receiving the atrial cardioverting electrical energy. A second warning may optionally be given slightly before delivery of the cardioversion pulse, if desired. If the patient does not perceive the analgesic effect of the pain alleviating therapy during the warning delay, the patient may use the programmer 322 to reset the delay timer 565 to delay delivery of the cardioversion pulse until timer 565 expires. Alternatively, the programmer may instead allow the patient to delay delivery of the pulse until the patient perceives the analgesic effect, and allow delivery of the cardioversion therapy only following a patient initiated enable signal to the implanted device indicating that therapy may be delivered. As yet another alternative, the programmer may be employed to simply abort the therapy during the warning delay, which may be especially useful if therapy was initially requested by the patient, and the patient's symptoms have subsided.

A warning system as described above, including apparatus specifically dedicated to providing the warning may not be necessary if the patient can independently feel the analgesic take effect.

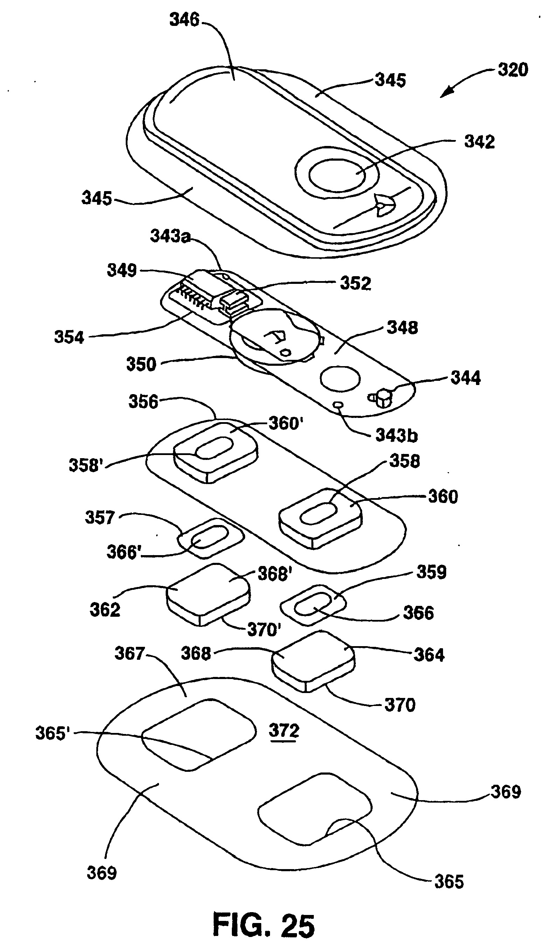

FIG. 25 illustrates a representative electrotransport delivery device that may be used in conjunction with the present invention. Device 320 comprises an upper housing 346, a circuit board assembly 348, a lower housing 356, anode electrode 357, cathode electrode 359, anode reservoir 362, cathode reservoir 364 and skin-compatible adhesive 367. Upper housing 346 has lateral wings 345, which assist in holding device 320 on a patient's skin. Upper housing 346 is preferably composed of an injection moldable elastomer (e.g., ethylene vinyl acetate). Printed circuit board assembly 348 comprises an integrated circuit 349 coupled to discrete components 352, antenna 354 and battery 350. Circuit board assembly 348 is attached to housing 346 by posts (not shown in figure 25) passing through openings 343a and 343b, the ends of the posts being heated/melted in order to heat stake the circuit board assembly 348 to the housing 346. Lower housing 356 is attached to the upper housing 346 by means of adhesive 367, the upper surface 372 of adhesive 367 being adhered to both lower housing 356 and upper housing 346 including the bottom surfaces of wings 345.

Shown (partially) on the underside of circuit board assembly 348 is a button cell battery 350. Other types of batteries may also be employed to power device 320.

The device 320 is generally comprised of battery 350, electronic circuitry 349, 352, 354, electrodes 357, 359, and hydrogel drug reservoirs 362, 364, all of which are integrated into a self-contained unit. The outputs (not shown in Fig. 25) of the circuit board assembly 348 make electrical contact with the electrodes 359, and 357 through openings 358, 358' in the depressions 360, 360' formed in lower housing 356, by means of electrically conductive adhesive strips 366, 366'. Electrodes 357 and 359, in turn, are in direct mechanical and electrical contact with the top sides 368, 368' of drug reservoirs 362 and 364. The bottom sides 370', 370 of drug reservoirs 362, 364 contact the patient's skin through the openings 365', 365 in adhesive 367.

Device 320 optionally has a feature, which allows the patient to self-administer a dose of drug by electrotransport. Upon depression of push button switch 342, the electronic circuitry on circuit board assembly 348 delivers a predetermined DC current to the electrode/reservoirs 357, 362 and 359, 364 for a delivery interval of predetermined length. The push button switch 342 is conveniently located on the top side of device 320 and is easily actuated through clothing. A double press of the push button switch 342 within a short time period, e.g., three seconds, is preferably used to activate the device for delivery of drug, thereby minimizing the likelihood of inadvertent activation of the device 320. Preferably, the device transmits to the user a visual, tactile and/or audible confirmation of the onset of the drug delivery interval by means of LED 344 becoming lit, TENs-like stimulation via electrodes 357 and 359 and/or an audible sound signal from, e.g., a "beeper". Drug is delivered through the patient's skin by electrotransport, e.g., on the arm or body, over the predetermined delivery interval.

Anodic electrode 357 is preferably comprised of silver and cathodic electrode 359 is preferably comprised of silver chloride. Both reservoirs 362 and 364 are preferably comprised of polymeric gel materials. Electrodes 357, 359 and reservoirs 362, 364 are retained by lower housing 356.

A liquid drug solution or suspension is contained in at least one of the reservoirs 362 and 364. Drug concentrations in the range of approximately $1 \times 10^{-4}$ M to 1.0 M or more can be used, with drug concentrations, in the lower portion of the range being preferred. Typically, the reservoir containing the drug will also contain the selected countersensitizing agent, in an amount and concentration effective to provide the flux necessary to reduce or prevent sensitization of the skin or mucosa.

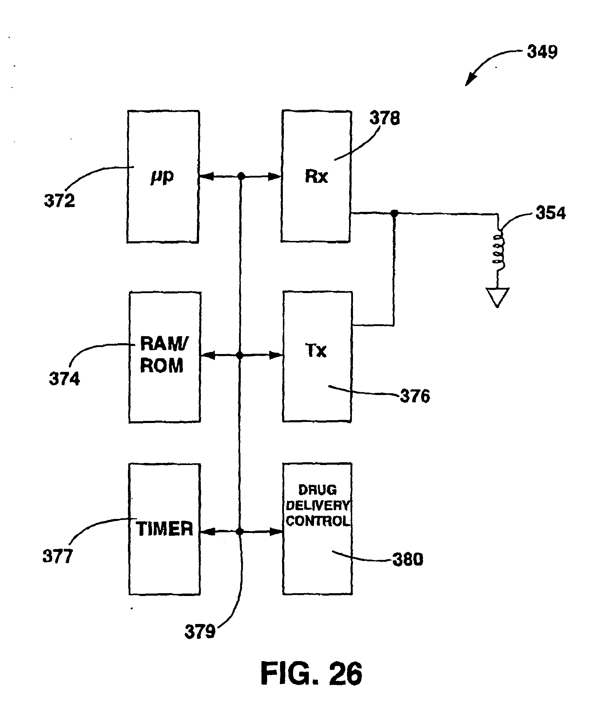

FIG. 26 shows a simplified schematic block diagram of drug delivery system 320 integrated circuit 349. Microprocessor 372 under control of a program contained in RAM/ROM 374 (interconnected via bi-directional bus 379) receives commands from IMD 312 through receiver 378 and antenna 354. Upon a command to initiate drug delivery, the microprocessor 372 initiates drug delivery through drug delivery control block 380, which initiates iontophoretic drug delivery through two electrodes (not shown). Timer 377 times the length of time for drug delivery under control of intervals stored in RAM/ROM 374 previously programmed via programmer 322 (not shown in figure 26). Upon timer 377 time out, a signal is sent to IMD 312 from transmitter block 376 and antenna 354 using the same telemetry system described herein above.

The infusion of various analgesic drugs or agents (or, simply "analgesics") including opiates (i.e. morphine sulfate, hydromorphone) and non-opiates (i.e. alpha-2 adrenergic agonists and neuron specific calcium channel blocking agents) have demonstrated rapid and effective analgesia following administration. Dependent upon the specific analgesic administered, it is also reported that the onset of pain suppression occurs in a couple of minutes to one hour, and the duration of analgesia may range from 4-24 hours. The delay in analgesia onset is not problematic, since rapid cardioversion is not necessary for atrial fibrillation as opposed to ventricular fibrillation. Time to analgesia can be utilized by the system 330 to re-verify the continuation of atrial fibrillation, charge storage capacitors to deliver the cardioversion shock, and ensure ventricular sensing to allow cardioversion shock synchronization with the R-wave of the cardiac cycle.

A first alternative embodiment of the invention may employ the drug delivery system 320 for delivery of a cardioversion or defibrillation threshold reducing agent such as D-sotalol, Procainamide or Quinidine as an alternative to, or in conjunction with, delivery of the pain alleviating therapy discussed above. The reduction of defibrillation threshold in such case would provide the possibility of a reduced amplitude, less painful cardioversion pulse. The delivery of a threshold reducing agent thus can be employed as a pain alleviating therapy or as part of a pain alleviating therapy. In a more complex embodiment, two separate drug delivery systems might be employed to allow delivery of the threshold reducing agent alone or in conjunction with an analgesic.

A second alternative embodiment of the invention may employ the drug delivery system 320 for delivery of diuretic and blood pressure regulating agents such as Thiazide diuretics (hydrochlorothiazide, chlorthalidone), usually adequate for mild heart failure, loop diuretics (furosemide, bumetanide, ethacrynic acid) reserved for severe volume overload or thiazide-resistant edema. Additionally, ACE inhibitors have been shown to prevent or slow the progression of heart failure in patients with symptomatic and asymptomatic left ventricular dysfunction. Currently, four agents are used for the treatment of CHF (Congestive Heart Failure): captopril, enalapril, lisinopril, and quinapril. The implementation of IMD 312 of this embodiment would use the Medtronic Chronicle™ CHF monitoring system such as described in US Patent Nos. 5,535,752 and 6,155,267, The IMD 312, as described in the '267 patent, would monitor chronic data representative of at least one physiological parameter. The chronic data is monitored to detect changes in state of the at least one physiological parameter. Data associated with detected changes in state is stored within IMD 312. The detection of changes in state of the at least one physiological parameter is performed by establishing a baseline (e.g., a center reference line and upper and lower control limits), and then determining if the chronic data being monitored satisfies predetermined, preprogrammed conditions (e.g., conditions based on the center reference line and the upper and lower control limits) indicative of a change in state of the at least one physiological parameter. If data occurs outside of these predetermined limits, the IMD 312 sends telemetry signals to drug delivery system 320 to initiate the delivery of the appropriate drug as described herein above.

A third alternative embodiment may utilize the drug delivery system 320 as the patient activator/programmer 322 by incorporating the features and function as described in US Patent No. 5,987,356.

FIG. 27 depicts a flow chart of an operation of the system shown in figure 24. At step S100, which continues at all times (except during the delivery of atrial cardioversion shock), atrial activity of the heart is sensed. At step 102, the atrial fibrillation detection algorithm is invoked in atrial fibrillation detector 570. If it is detected, then in step S104, the IMD 330 transmits a signal to the drug delivery system 320 via drug delivery control 590, RF transmitter 592 and antenna 594 to initiate delivery at S105 of a programmed bolus of the pain alleviating drug from drug dispenser 320.

The charge delivery control 572 may be commanded to start charge up of the charge storage capacitors, but it is preferred to delay capacitor charge up until the end of the delay for the analgesic to take effect and commence capacitor charge up during the analgesic time course, that is, the time period that the analgesia effect is expected to continue. At S107 the patch 520 transmits a confirmation of drug delivery completion back to the IMD 312. Optional warning steps for the patients to show the status of the detection, drug delivery and cardioverter status may be included but are not shown in the flow chart of Fig. 7.

A delay timer 565 is loaded and enabled to time out the delay for the analgesia effect to take place in steps S106 and S108. During this delay, the continuation of the atrial fibrillation episode may be verified in steps S100 and S102 and the algorithm may optionally be halted at that point. However, since atrial fibrillation bouts reoccur and since the bolus of pain alleviating drug is already delivered, re-confirmation at the end of the delay times is sufficient to determine whether or not to deliver the cardioversion shock therapy.

When the delay timer 565 times out in step S108, the delay timer 565 is reset for the analgesic time course and is started in step S110. The atrial fibrillation detection is re-verified in step S112 during the analgesic time course. If it only re-verified after the analgesic time course times out, then it is necessary to repeat steps S104-S114 until it is re-verified during an analgesic time course.

Then, in step S116, a charge delivery control 572 is commanded to enable the storage capacitor charge circuit 574 to charge the high voltage output capacitors up to the cardioversion energy set in level stage 569. The microcomputer 562 then sets a synchronization time interval in interval set stage 566 from an R-wave detected by R-wave detector 552. The ventricular timer 564 then provides a blanking signal to the ventricular and atrial sense amplifiers 550 and 554. Both operations may be performed in step S118. Re-verification of continued atrial fibrillation may also be performed between steps S116 and S118.

At the expiration of the synchronization time interval in ventricular timer 564, a command is applied through the charge delivery control to operate a discharge circuit 576 to discharge the atrial cardioversion shock via electrodes 544 and 546. After the atrial cardioversion shock is delivered, the atrial and ventricular sense amplifiers are again enabled, and the presence or absence of atrial fibrillation is again tested in step S 112. If the episode is terminated, then the algorithm loops back to step S100.

If the episode is not terminated, the steps of figure 27 may be repeated. After a certain number of attempts, the available therapies may be exhausted. Whether or not the therapies are successful, the patient will likely have been advised to contact the attending physician. The event history of the episodes and delivered therapies are recorded in RAM 582 for subsequent telemetry out and analysis by the physician in a manner well known in the art in order to assist in reprogramming therapies.

Thus, the methodological sequence to provide the pain alleviating therapy to counter the pain induced by delivery of atrial cardioversion energy includes the initial detection of atrial fibrillation, optional warning to the patient, drug infusion therapy to produce analgesia, time out to allow analgesia to take effect and the charge storage capacitors to be charged, reverification of atrial fibrillation, delivery of the cardioversion energy, and verification that successful atrial defibrillation has taken place. Should successful atrial cardioversion not take place, the steps of figure 27, would be reinitiated, except that analgesic drug delivery would not be repeated unless the analgesic time course time had timed out in order to prevent drug overdose.

Depending on the analgesic employed, it may also be desirable to include a further timer to inhibit delivery of a further analgesic bolus timed from the previous delivery for a further time delay to prevent drug overdose. Such a timer may take into account the cumulative amount of analgesic delivered over a set time period.

In addition, it may be desirable to provide the patient with the option of using programmer 322 to temporarily program the delivery of an increased quantity of analgesic, if the desired analgesia effect is not achieved at the permanently programmed setting. A time and date record of such patient programmed increases may be kept in the system memory for review by the physician, and the repetitive use of the programmer may be inhibited.

A physiologic monitoring system consisting of an implantable therapeutic device (e.g., pacemaker or ICD) and an externally-applied external monitor incorporating: 1) electrodes for detecting the electrical behavior of the implanted device, 2) a controller, 3) a power source, and 4) a radio transmitter for sending information to an external receiving system. The external monitoring system is constantly monitoring the therapeutic electrical output of the implantable device and decoding the electrical behavior according to pre-set algorithms to uniquely infer what the implanted device is sensing. The radio transmitter in the external monitor then sends the decoded data to an external receiver, which subsequently sends it on to a patient monitoring system. This invention allows an external monitor to receive physiologic data without using telemetry simply by monitoring the easily-detected electrical therapy (e.g., pacing or high voltage cardioversion/defibrillation delivered by the implanted device.

Implanted devices like pacemakers and ICDs are constantly sensing their associated physiological environment. For example, pacemakers and ICD's are always sensing native electrical activity in the atria and ventricles, associated data such as activity or respiration, and derived parameters such as presence or absence of capture, lead integrity problems, and the like. In order to know what an implanted device is sensing, radio telemetry, typically in the 175 KHz range, is widely used to interrogate the memory in the device. This requires a sophisticated instrument such as a programmer which is capable of interacting with the implanted device along with computational capabilities to decode the data in the telemetry stream. Another method of determining what the device is sensing is to use a "Patient Alert" type feature, where the device actually generates an audible tone when certain sensed conditions are fulfilled (such as a lead problem). This requires the patient to listen for the tone, decide what the tone means, and contact a health care worker.

The following are examples of detection by the electrodes: 1) Detection of arrhythmias, 2) Detection of device malfunction, such as lead failure or misprogramming, 3) Detection of underlying physiologic parameters such as hemodynamics and other sensed parameters.

The therapeutic electrical output of a therapeutic implantable device like a pacemaker or an ICD is easily detectable from the skin surface using ECG electrodes and a standard ECG preamplifier which are well known in the art. The therapeutic electrical output from a pacemaker consists of pacing pulses in the 1-5 volt range, which can be detected by skin electrodes as several millivolt signals. The high voltage defibrillation output from an implantable defibrillator consists of ramp waveforms in the hundreds of volts range, which again can be easily detected as several volt signals on the skin surface. The therapeutic electrical output of a therapeutic implantable device occurs as a response to a set of sensed conditions. It is frequently possible to create a 1-1 mapping between a specific type of therapeutic electrical output and the sensed conditions that leads to that output, based on the characteristics of the specific implanted device and the way it is programmed. As a very simple example, ventricular pacing in a VVI pacemaker occurs when no intrinsic ventricular electrical signal is detected by the pacemaker during a pre-set time interval. Therefore, detecting a regular set of ventricular pacing stimuli can be taken as evidence that there are no intrinsic ventricular depolarizations occurring, without having to measure intrinsic ventricular activity independently. Implantable therapeutic devices have evolved considerably in complexity from VVI pacing, and there are now specific therapeutic algorithms built into most devices which occur in response to a particular set of sensed conditions. For example, Rate Drop Response is a particular type of pacing which is easily recognized by a rapid start of regular pacing, at a relatively high rate (such as 120 bpm), followed by a gradual fallback in pacing rate over the next several minutes. This particular algorithm is activated by a specific set of trigger criteria, including a fall in the intrinsic ventricular rate through a specific rate window over a specific time window. Therefore, the specific therapeutic electrical "signature" generated by the Rated Crop Response algorithm, which is easily detectable at the skin surface, allows the external device to determine that a specific type of intrinsic rate change occurred and to send this information to a patient monitoring system. Similarly, an ICD may be programmed to deliver a burst of autodecremental anti-tachycardia pacing (ATP) if a ventricular arrhythmia of a certain rate is detected. The external monitor can easily detect the specific pattern of auto-decremental ATP as a "signature" of a ventricular arrhythmia and, similarly, identify this arrhythmia and send the detection to an external patient monitoring system. There are many other examples of unique therapeutic electrical "signatures" which can be used by the external monitoring system to generate a specific diagnosis. In addition to detecting physiologic conditions such as arrhythmias, this device could also detect potential malfunctions or problems with the implanted device. For example, ventricular safety pacing occurs in response to a sensed beat during a pacemaker refractory period. The safety pace is easily recognizable by its characteristic coupling interval from the previous pace. Frequent ventricular safety pacing sometimes indicates a lead insulation problem. Therefore, the detection of frequent ventricular safety pacing could initiate a transmission of "possible ventricular lead problem." If the monitor also detected intrinsic electrical activity of the heart (e.g. easily detected ORS complexes), relationships between the therapeutic electrical output of the device and the detected intrinsic rhythm could add additional richness to the detection scheme. For example, a dissociation between ventricular pacing spikes and intrinsic ventricular beats could indicate loss of capture. Detecting this pattern could generate the message "possible loss of capture." Another potential use is to detect underlying physiologic conditions that the implanted device responds to. For example, future implantable therapeutic devices may incorporate hemodynamic sensors or ischemia sensors. It is conceivable that the implanted device may respond with a specific therapeutic electrical intervention, based on the data from these sensors. For example, an implantable pacemaker may be programmed to undergo a certain type of pacing rate fallback if ischemia is detected. This specific behavior could be decoded as indicative of sensed ischemia. Referring to FIG. 1, electrodes are applied to the patient (at left) and connected to the EKG preamplifier on the external monitoring system. The EKG preamplifier sends the amplified signal to the Algorithm Decoder. The Algorithm Decoder typically contains either an Analog to Digital Converter (ACD) or an analog sense amplifier with comparator circuitry in order to detect therapeutic electrical signals. The output from the ADC or comparator is then sequentially fed to the Algorithm Decodeer circuitry, which compares the incoming signal with a set of patterns stored in the Algorithm Storage RAM. The Algorithm Storage RAM contains the specific therapeutic patterns tat are used to decode the therapeutic electrical signals into specific patterns. This RAM would be customized for the specific implantable device and the specific programming; for example, VT at a certain rate might be programmed to cause the implantable device to initiate a specific type of ATP. This information would then be loaded into the Algorithm Storage RAM. The diagram shows how the radio transmitter could also function as a receiver to program the Algorithm Storage RAM. The Algorithm Storage RAM would also contain a PLA that translates each detected therapeutic electrical pattern into a simple signal that is then sent to the radio transmitter for transmission to the receiving system. As in the example above, autodecremental ATP in response to a detected VT might generate a "10001000" radio signal, while a defibrillation shock might general a "11111111" radio signal, which the receiver would decode into the appropriate sensed rhythm. In the example given above, other conditions such as excess ventricular safety pacing, dissociation between pacing and intrinsic beats, or a specif rate fallback behavior indicating ischemia would also generate unique identifying codes. Also noted in the diagram is a controller, which would typically consist of a microprocessor and its associated software or microcode required to operate the entire device. In addition, the figure demonstrates a power source (e.g., battery, which could be rechargeable), which supplies power to all of the electronics in the monitor.

One method of using the invention includes: 1) Using the therapeutic electrical output of an implanted device to discern sensed conditions that initiated the therapeutic electrical intervention. 2) Having an external cutaneous device detect the therapeutic electrical output of an implanted device that allows the external device to discern the sensed conditions of the implanted device. 3) Use of an algorithm decoder to translate therapeutic electrical stimuli into specific sensed conditions based on the specific characteristics of the implanted device and its associated programming. 4) Use of a radio link to download specific algorithm information into the algorithm decoder. 5) Detecting implantable device malfunction through external decoding of therapeutic electrical interventions. 6) Detecting underlying physiologic states (e.g., ischemia) through external decoding of therapeutic electrical interventions.

Thus, embodiments of the Implantable Medical Device And Patch System are disclosed. One skilled in the art will appreciate that the present invention can be practiced with embodiments other than those disclosed. The disclosed embodiments are presented for purposes of illustration and not limitation, and the present invention is limited only by the claims that follow.

**Claims**

1. A drug delivery patch (130) capable of administering a therapeutic substance to a patient having skin based on therapeutic action taken by an implanted medical device, the patch comprising:

   a housing (141) configured for attachment to the patient so that the housing contacts the skin of the patient;
   a therapeutic substance reservoir (142) coupled to the housing (141), the therapeutic substance reservoir having a reservoir outlet (152);
   sensing means for sensing the therapeutic action taken by the implanted medical device by sensing as opposed to telemetry;
   control means for controlling the functions of the patch through a control signal wherein the control signal is based at least in part on the sensed action and
   delivery means, operatively coupled to the reservoir outlet, for delivering a metered amount of the therapeutic substance to the patient in response to the control signal; and wherein the therapeutic action taken by the implanted medical device comprises generating a therapeutic electrical signal, wherein the sensing means senses the therapeutic electrical signal by sensing as opposed to telemetry and wherein the control means controls the function of the patch, at least in part, based on the sensed therapeutic electrical signal.

2. A drug delivery patch as in claim 1 wherein the patch (130) is adhered to the skin of the patient.

3. A drug delivery patch as in claim 1 wherein the patch (130) is a transdermal drug delivery patch.

4. A drug delivery patch (130) as in claim 3 wherein the transdermal drug delivery patch comprises an iontophoretic transdermal drug delivery patch.

5. A drug delivery patch (130) as in claim 3 wherein the transdermal drug delivery patch comprises a sonophoretic transdermal drug delivery patch.

6. A drug delivery patch (130) as in claim 3 wherein the transdermal drug delivery patch utilizes a laser to assist in transmission of the drug through the skin of the patient.

7. A drug delivery patch (130) as in claim 3 wherein the transdermal drug delivery patch utilizes a needle (154) to administer the therapeutic substance to the patient.

8. A drug delivery patch (130) as in claim 1 wherein the therapeutic substance is selected from the group consisting of diuretic drugs, beta blockers, vasodilators, Phenytoin, Conantonkin-G, glucagons, morphine, hydromorphone and naloxone.

9. A therapeutic substance delivery system for delivery of a therapeutic substance into a human body using a drug delivery patch as in claim 1 and additionally comprising an implanted medical device capable of generating the therapeutic action to the human body for therapeutic reasons.

10. A therapeutic substance delivery system as in claim 9 wherein the therapeutic action taken by the implanted medical device comprises generating a therapeutic electrical signal, wherein the sensing means senses the therapeutic electrical signal and wherein the control means controls the functions of the patch, at least in part, based on the sensed therapeutic electrical signal.


**Patentansprüche**

1. Arzneimittelzuführungspflaster (130), das dazu in der Lage ist, basierend auf einer therapeutischen Handlung, die von einer implantierten medizinischen Vorrichtung vorgenommen ist, einem Patienten mit Haut eine therapeutische Substanz zu verabreichen, mit:

   einem Gehäuse (141), das zur Befestigung an dem Patienten konfiguriert ist, so dass das Gehäuse die Haut des Patienten berührt,
   einem Vorratsbehälter (142) für die therapeutische Substanz, der mit dem Gehäuse (141) gekoppelt ist, wobei der Vorratsbehälter für die therapeutische Substanz einen Vorratsbehälterauslass (152) aufweist,

Sensormitteln zum Wahrnehmen der von der implantierten medizinischen Vorrichtung vorgenommenen therapeutischen Handlung mittels Wahrnehmung im Gegensatz zu Telemetrie,
Steuerungsmitteln zum Steuern der Funktionen des Pflasters mittels eines Steuerungssignals, wobei das Steuerungssignal zumindest zum Teil auf der wahrgenommenen Handlung basiert, und
Zuführungsmitteln, die wirksam mit dem Vorratsbehälterauslass gekoppelt sind, zum Zuführen einer dosierten Menge der therapeutischen Substanz zu dem Patienten in Reaktion auf das Steuerungssignal, und wobei die von der implantierten medizinischen Vorrichtung vorgenommene therapeutische Handlung das Erzeugen eines therapeutischen elektrischen Signals umfasst, wobei die Sensormittel das therapeutische elektrische Signal durch Wahrnehmung im Gegensatz zu Telemetrie wahrnehmen und wobei die Steuerungsmittel die Funktion des Pflasters zumindest zum Teil basierend auf dem wahrgenommenen therapeutischen elektrischen Signal steuern.

2. Arzneimittelzuführungspflaster nach Anspruch 1, bei dem das Pflaster (130) auf die Haut des Patienten aufgeklebt ist.

3. Arzneimittelzuführungspflaster nach Anspruch 1, bei dem das Pflaster (130) ein transdermales Arzneimittelzuführungspflaster ist.

4. Arzneimittelzuführungspflaster (130) nach Anspruch 3, bei dem das transdermale Arzneimittelzuführungspflaster ein iontophoretisches transdermales Arzneimittelzuführungspflaster umfasst.

5. Arzneimittelzuführungspflaster (130) nach Anspruch 3, bei dem das transdermale Arzneimittelzuführungspflaster ein sonophoretisches transdermales Arzneimittelzuführungspflaster umfasst.

6. Arzneimittelzuführungspflaster (130) nach Anspruch 3, bei dem das transdermale Arzneimittelzuführungspflaster einen Laser dazu verwendet, bei der Überführung des Arzneimittels durch die Haut des Patienten zu helfen.

7. Arzneimittelzuführungspflaster (130) nach Anspruch 3, bei dem das transdermale Arzneimittelzuführungspflaster eine Nadel (154) dazu verwendet, dem Patienten die therapeutische Substanz zu verabreichen.

8. Arzneimittelzuführungspflaster (130) nach Anspruch 1, bei dem die therapeutische Substanz aus der aus diuretischen Arzneimitteln, Betablockern, Vasodilatoren, Phenytoin, Conantokin-G, Glucagonen, Morphium, Hydromorphon und Naloxon bestehenden Gruppe ausgewählt ist.

9. Zuführungssystem für eine therapeutische Substanz zum Zuführen einer therapeutischen Substanz in einen menschlichen Körper unter Verwendung eines Arzneimittelzuführungspflasters nach Anspruch 1, das zusätzlich eine implantierte medizinische Vorrichtung umfasst, die dazu in der Lage ist, die therapeutische Handlung an dem menschlichen Körper zu therapeutischen Zwecken zu erzeugen.

10. Zuführungssystem für eine therapeutische Substanz nach Anspruch 9, bei dem die von der implantierten medizinischen Vorrichtung vorgenommene therapeutische Handlung das Erzeugen eines therapeutischen elektrischen Signals umfasst, wobei die Sensormittel das therapeutische elektrische Signal wahrnehmen und wobei das Steuerungsmittel die Funktionen des Pflasters zumindest zum Teil basierend auf dem wahrgenommenen therapeutischen elektrischen Signal steuern.

**Revendications**

1. Timbre d'administration de médicament (130) capable d'administrer une substance thérapeutique à un patient ayant une peau sur la base d'une action thérapeutique réalisée par un dispositif médical implanté, le timbre comportant :

une enceinte (141) configurée pour être fixée au patient de sorte que l'enceinte vient au contact de la peau du patient,
un réservoir de substance thérapeutique (142) couplé à l'enceinte (141), le réservoir de substance thérapeutique ayant une sortie de réservoir (152),
des moyens de détection pour détecter l'action thérapeutique réalisée par le dispositif médical implanté par une détection opposée à une télémétrie,
des moyens de commande pour commander les fonctions du timbre par l'intermédiaire d'un signal de commande, le signal de commande étant basé au moins en partie sur l'action détectée, et

des moyens d'administration, couplés de manière opérationnelle à la sortie de réservoir, pour administrer une quantité mesurée de la substance thérapeutique au patient en réponse au signal de commande, et dans lequel l'action thérapeutique réalisée par le dispositif médical implanté comporte la génération d'un signal électrique thérapeutique, les moyens de détection détectant le signal électrique thérapeutique par une détection opposée à une télémétrie et les moyens de commande commandant la fonction du timbre, au moins en partie, sur la base du signal électrique thérapeutique détecté.

**2.** Timbre d'administration de médicament selon la revendication 1, dans lequel le timbre (130) est collé à la peau du patient.

**3.** Timbre d'administration de médicament selon la revendication 1, dans lequel le timbre (130) est un timbre d'administration de médicament transdermique.

**4.** Timbre d'administration de médicament (130) selon la revendication 3, dans lequel le timbre d'administration de médicament transdermique comporte un timbre d'administration de médicament transdermique iontophorétique.

**5.** Timbre d'administration de médicament (130) selon la revendication 3, dans lequel le timbre d'administration de médicament transdermique comporte un timbre d'administration de médicament transdermique sonophorétique.

**6.** Timbre d'administration de médicament (130) selon la revendication 3, dans lequel le timbre d'administration de médicament transdermique utilise un laser pour aider à la transmission du médicament à travers la peau du patient.

**7.** Timbre d'administration de médicament (130) selon la revendication 3 dans lequel le timbre d'administration de médicament transdermique utilise une aiguille (154) pour administrer la substance thérapeutique au patient.

**8.** Timbre d'administration de médicament (130) selon la revendication 1, dans lequel la substance thérapeutique est sélectionnée parmi le groupe constitué de médicament diurétiques, de bêta-bloquants, de vasodilatateurs, de Phénytoïne, de Conantokine-G, de glucagons, de morphine, d'hydromorphone et de naloxone.

**9.** Système d'administration de substance thérapeutique pour administrer une substance thérapeutique dans un corps humain en utilisant un timbre d'administration de médicament selon la revendication 1 et comportant de plus un dispositif médical implanté capable de générer l'action thérapeutique sur le corps humain pour des raisons thérapeutiques.

**10.** Système d'administration de substance thérapeutique selon la revendication 9, dans lequel l'action thérapeutique réalisée par le dispositif médical implanté comporte la génération d'un signal électrique thérapeutique, dans lequel les moyens de détection détectent le signal électrique thérapeutique et dans lequel les moyens de commande commandent les fonctions du timbre, au moins en partie, sur la base du signal électrique thérapeutique détecté.

# FIG. 1

**40**

Indicator

External Patch Device

32

| Delivery | 38 |
| Control | 36 |
| Communication | 34 |
| Sensor | 33 |

Outside World

Environmental Interaction

Internet Controlled

E-Bus

PAC

Physician

Store Outcome Data

Implantable Medical Device

30

| Implant Device | |
| 31 | Sensor |

EP 1 385 570 B1

EP 1 385 570 B1

# FIG. 2

**45**
External Infusion
Patch (including
telemetry)

| | |
|---|---|
| **Antenna** | 47 |
| **IC** | 49 |
| **Battery** | 51 |
| **Valve** | 53 |
| **Drug Reservoir** | 55 |
| **Drug Propulsion Means** | 57 |

# FIG. 3

**Smart Infusion Patch**

| | |
|---|---|
| Communication Block | Power Management |
| Sensing Block | Patient Control Block |
| Therapy Control | Overhead Blocks 77 |

67 — Communication Block

73 — Power Management

69 — Sensing Block

75 — Patient Control Block

71

59

**Implanted Medical Device**

| | |
|---|---|
| Therapeutic Block 63 | Communication Block 65 |

61

EP 1 385 570 B1

FIG. 4

EP 1 385 570 B1

FIG. 5

FIG. 6

FIG. 7

FIG. 8

**FIG. 9**

EP 1 385 570 B1

**FIG. 10**

EP 1 385 570 B1

**FIG. 11**

EP 1 385 570 B1

**FIG. 12**

176

186

168

166

170

188

154

**FIG. 13**

148

184

162

166

**FIG. 14a**

148

184

162

**FIG. 14b**

EP 1 385 570 B1

190

202

204

200

## FIG. 15

EP 1 385 570 B1

Yoke ⟶ 200

170 ⟶ Coil A | Coil B ⟶ 168

166

194 ⟶ S | N ⟶ 192

170 ⟶ | ⟶ 168

Magnet ⟶ 200

**FIG. 16**

## FIG. 17

Two Coils and One Magnet

EP 1 385 570 B1

**FIG. 18**

EP 1 385 570 B1

EP 1 385 570 B1

Yoke

~200

170 — **Coil A** **Coil B** ~171 **Coil A** — 168

202, 206 — 102

**Poles** → | S | N | N | S | ~202, 204

170 — 171 — 168

Magnets ~200

# FIG. 19

Three Coils and Two Magnets

212 — ENERGIZING COILS ◄— PROGRAMING ELECTRONICS — 208

214 — RETRACTING PISTON ◄— SUPPLYING THERAPEUTIC SUBSTANCE — 210

216 — OPENING INLET VALVE

218 — LOADING BIASING ELEMENT

220 — FILLING PUMP

222 — DE-ENERGIZING COILS

224 — UNLOADING BIASING ELEMENT

226 — ACTUATING PISTON

228 — OPENING OUTLET VALVE

230 — EXPULSING STROKE —► DELIVERING THERAPEUTIC SUBSTANCE — 232

**FIG. 20**

FIG. 21

**FIG. 22**

FIG. 23

**FIG. 24**

FIG. 25

FIG. 26

FIG. 27